# EUROPEAN PATENT SPECIFICATION

(11) **EP 2 193 208 B1**
(45) Date of publication and mention of the grant of the patent: **20.04.2016**
(21) Application number: 08800088.0
(22) Date of filing: 02.10.2008
(51) Int. Cl.: C12Q 1/68

(54) **A METHOD OF DNA AMPLIFICATION**
DNA-AMPLIFIKATIONSVERFAHREN
PROCÉDÉ D'AMPLIFICATION D'ADN

(30) Priority: 22.10.2007 US 981761 P
(43) Date of publication of application: 09.06.2010
(73) Proprietor: Monoquant PTY LTD, South Australia 5000 (AU)
(72) Inventor: MORLEY, Alexander, Alan, Glenelg South Australia 5045 (AU)
(74) Representative: Wilkins, Christopher George
(86) International application number: PCT/AU2008/001453
(87) International publication number: WO 2009/052547

(56) References cited:
- WO-A1-2006/094360
- ZHU LING-XIANG ET AL: "Multiplex asymmetric PCR-based oligonucleotide microarray for detection of drug resistance genes containing single mutations in Enterobacteriaceae", ANTIMICROBIAL AGENTS AND CHEMOTHERAPY, AMERICAN SOCIETY FOR MICROBIOLOGY, WASHINGTON, DC, US, vol. 51, no. 10, 1 October 2007 (2007-10-01), pages 3707-3713, XP002532643, ISSN: 0066-4804, DOI: DOI:10.1128/AAC.01461-06 [retrieved on 2007-07-23]
- MEYERHANS, A. ET AL.: 'Strand specific PCR amplification of low copy number DNA.' NUC. ACID RES. vol. 20, no. 3, 1992, pages 521 - 523, XP001525386
- METZLER, M. ET AL.: 'Asymmetric multiplex-polymerase chain reaction - a high throughput method for detection and sequencing genomic fusion sites in t(4;11).' BR. J. HAEMATOL. vol. 124, 2004, pages 47 - 54, XP008122029
- OETTING, W.S. ET AL.: 'Multiplexed short tandem repeat polymorphisms of the Weber 8A set of markers using tailed primers and infrared fluorescence detection.' ELECTROPHORESIS vol. 19, 1998, pages 3079 - 3083, XP008132185

### SEQUENCE LISTING

<110> MONOQUANT PTY. LTD.
   MORLEY, ALEXANDER ALAN
<120> A METHOD OF DNA AMPLIFICATION
<130> 30635146/TDO
<150> US 60/981761
   <151> 2007-10-22
<160> 398
<170> Patent In version 3.4
<210> 1
   <211> 25
   <212> DNA
   <213> synthetic
<220>
   <221> misc_feature
   <222> (16)..(20)
   <223> n is a, c, g, or t
<400> 1
   tgctaggatc caaggnnnnn attcg 25
<210> 2
   <211> 53
   <212> DNA
   <213> synthetic
<400> 2
   tagccttggt ccacactgag cactgggttc aagggcarag cagggaaacc tca 53
<210> 3
   <211> 52
   <212> DNA
   <213> synthetic
<400> 3
   tcagcactta tcagaaactt ttgacaatat agacaattta agtcccaagg ca 52
<210> 4
   <211> 52
   <212> DNA
   <213> synthetic
<400> 4
   tacccagccg amctagccca taaaatacat agtgcaaatc wtatggatga ta 52
<210> 5
   <211> 52
   <212> DNA
   <213> synthetic
<400> 5
   cacagaaaca gtcattttca ttctcaaaga gttcatctgg acaaagcagt aa 52
<210> 6
   <211> 23
   <212> DNA
   <213> synthetic
<400> 6
   gcaacactgt gacgtactgg agg 23
<210> 7
   <211> 23
   <212> DNA
   <213> synthetic
<400> 7
   gcaacactgt gacgtactgg agg 23
<210> 8
   <211> 46
   <212> DNA
   <213> synthetic
<400> 8
   tcgcttctag tctgtgccac agggcaacac tgtgacgtac tggagg 46
<210> 9
   <211> 23
   <212> DNA
   <213> synthetic
<400> 9
   tcgcttctag tctgtgccac agg 23
<210> 10
   <211> 45
   <212> DNA
   <213> synthetic
<400> 10
   gacatgtgct ggagatatga cgtcgcttct agtctgtgcc acagg 45
<210> 11
   <211> 22
   <212> DNA
   <213> synthetic
<400> 11
   gacatgtgct ggagatatga cg 22
<210> 12
   <211> 44
   <212> DNA
   <213> synthetic
<400> 12
   gagaaggtga tgcaacatac gggacatgtg ctggagatat gacg 44
<210> 13
   <211> 22
   <212> DNA
   <213> synthetic
<400> 13
   gagaaggtga tgcaacatac gg 22
<210> 14
   <211> 22
   <212> DNA
   <213> synthetic
<400> 14
   gcagtacaaa caacgcacag cg 22
<210> 15
   <211> 22
   <212> DNA
   <213> synthetic
<400> 15
   gcagtacaaa caacgcacag cg 22
<210> 16
   <211> 43
   <212> DNA
   <213> synthetic
<400> 16
   gccatctaca ttgccctgtc cgcagtacaa acaacgcaca gcg 43
<210> 17
   <211> 21
   <212> DNA
   <213> synthetic
<400> 17
   gccatctaca ttgccctgtc c 21
<210> 18
   <211> 41
   <212> DNA
   <213> synthetic
<400> 18
   gttgagacgt ggcaggaacg gccatctaca ttgccctgtc c 41
<210> 19
   <211> 20
   <212> DNA
   <213> synthetic
<400> 19
   gttgagacgt ggcaggaacg 20
<210> 20
   <211> 44
   <212> DNA
   <213> synthetic
<400> 20
   catgcgatta ttgcactctc ttccgttgag acgtggcagg aacg 44
<210> 21
   <211> 24
   <212> DNA
   <213> synthetic
<400> 21
   catgcgatta ttgcactctc ttcc 24
<210> 22
   <211> 25
   <212> DNA
   <213> synthetic
<220>
   <221> misc_feature
   <222> (16)..(20)
   <223> n is a, c, g, or t
<400> 22
   tgctaggatc caaggnnnnn attcg 25
<210> 23
   <211> 18
   <212> DNA
   <213> synthetic
<400> 23
   tcatgctagg atccaagg 18
<210> 24
   <211> 40
   <212> DNA
   <213> synthetic
<400> 24
   gcagttcatg gtcccgtcat cctcatgcta ggatccaagg 40
<210> 25
   <211> 22
   <212> DNA
   <213> synthetic
<400> 25
   gcagttcatg gtcccgtcat cc 22
<210> 26
   <211> 44
   <212> DNA
   <213> synthetic
<400> 26
   aaagaagagt cgcagaggct cggcagttca tggtcccgtc atcc 44
<210> 27
   <211> 22
   <212> DNA
   <213> synthetic
<400> 27
   aaagaagagt cgcagaggct cg 22
<210> 28
   <211> 44
   <212> DNA
   <213> synthetic
<400> 28
   tccttccttg gcaacgacat cgaaagaaga gtcgcagagg ctcg 44
<210> 29
   <211> 22
   <212> DNA
   <213> synthetic
<400> 29
   tccttccttg gcaacgacat cg 22
<210> 30
   <211> 20
   <212> DNA
   <213> synthetic
<400> 30
   cttctccctg acatccgtgg 20
<210> 31
   <211> 24
   <212> DNA
   <213> synthetic
<400> 31
   tgacatccgt ggagctgcag atgc 24
<210> 32
   <211> 47
   <212> DNA
   <213> synthetic
<400> 32
   tggcacgtga tcgtagagtg agggagctgc agatgctgac caactcg 47
<210> 33
   <211> 46
   <212> DNA
   <213> synthetic
<400> 33
   cggcatcctt gtcactgact gctgaccaac tcgtgtgtga aactcc 46
<210> 34
   <211> 21
   <212> DNA
   <213> synthetic
<400> 34
   ccagccctcc tctcctccag c 21
<210> 35
   <211> 23
   <212> DNA
   <213> synthetic
<400> 35
   ctctcctcca gctacctgcc agc 23
<210> 36
   <211> 45
   <212> DNA
   <213> synthetic
<400> 36
   tggcacgtga tcgtagagtg aggcagccgg cacttttggt caagc 45
<210> 37
   <211> 47
   <212> DNA
   <213> synthetic
<400> 37
   cggcatcctt gtcactgact gcggcacttt tggtcaagct gttttgc 47
<210> 38
   <211> 22
   <212> DNA
   <213> synthetic
<400> 38
   gaggttgttc agatgaccac gg 22
<210> 39
   <211> 24
   <212> DNA
   <213> synthetic
<400> 39
   accacgggac acctttgacc ctgg 24
<210> 40
   <211> 47
   <212> DNA
   <213> synthetic
<400> 40
   tggcacgtga tcgtagagtg aggcaccttt gaccctggcc gctgtgg 47
<210> 41
   <211> 46
   <212> DNA
   <213> synthetic
<400> 41
   cggcatcctt gtcactgact gcctggccgc tgtggagtgt ttgtgc 46
<210> 42
   <211> 24
   <212> DNA
   <213> synthetic
<400> 42
   cagctactgg agctgtcaga acag 24
<210> 43
   <211> 24
   <212> DNA
   <213> synthetic
<400> 43
   ctggagctgt cagaacagtg aagg 24
<210> 44
   <211> 47
   <212> DNA
   <213> synthetic
<400> 44
   tggcacgtga tcgtagagtg aggcagtgaa ggctggtaac acatgag 47
<210> 45
   <211> 46
   <212> DNA
   <213> synthetic
<400> 45
   cggcatcctt gtcactgact gctggtaaca catgagttgc actgtg 46
<210> 46
   <211> 17
   <212> DNA
   <213> synthetic
<400> 46
   tgggcctccc tgcatcc 17
<210> 47
   <211> 24
   <212> DNA
   <213> synthetic
<400> 47
   tccctgcatc cctgcatctc ctcc 24
<210> 48
   <211> 47
   <212> DNA
   <213> synthetic
<400> 48
   tggcacgtga tcgtagagtg aggatccctg catctcctcc cgggtcc 47
<210> 49
   <211> 46
   <212> DNA
   <213> synthetic
<400> 49
   cggcatcctt gtcactgact gctcctcccg ggtcctgtct gtgagc 46
<210> 50
   <211> 17
   <212> DNA
   <213> synthetic
<400> 50
   tccccctgca ccccacg 17
<210> 51
   <211> 24
   <212> DNA
   <213> synthetic
<400> 51
   cccacgactt ctccagcact gagc 24
<210> 52
   <211> 47
   <212> DNA
   <213> synthetic
<400> 52
   tggcacgtga tcgtagagtg aggccagcac tgagctgctt cctgtgc 47
<210> 53
   <211> 46
   <212> DNA
   <213> synthetic
<400> 53
   cggcatcctt gtcactgact gcgctgcttc ctgtgcccca cagtgg 46
<210> 54
   <211> 23
   <212> DNA
   <213> synthetic
<400> 54
   tggcacgtga tcgtagagtg agg 23
<210> 55
   <211> 22
   <212> DNA
   <213> synthetic
<400> 55
   cggcatcctt gtcactgact gc 22
<210> 56
   <211> 49
   <212> DNA
   <213> synthetic
<400> 56
   gcaacactgt gacgtactgg agggtctatc taaaattcac aaggaatgc 49
<210> 57
   <211> 47
   <212> DNA
   <213> synthetic
<400> 57
   gcaacactgt gacgtactgg aggaggcaaa gtaaaatcca agcaccc 47
<210> 58
   <211> 44
   <212> DNA
   <213> synthetic
<400> 58
   gcaacactgt gacgtactgg aggcactcct gcactccagc ctgg 44
<210> 59
   <211> 47
   <212> DNA
   <213> synthetic
<400> 59
   gcaacactgt gacgtactgg aggcaaccac caaagtgctt ttcctgg 47
<210> 60
   <211> 49
   <212> DNA
   <213> synthetic
<400> 60
   gcaacactgt gacgtactgg aggatatggc atctgtaaat attaccacc 49
<210> 61
   <211> 44
   <212> DNA
   <213> synthetic
<400> 61
   gcaacactgt gacgtactgg aggtgcctcg gcctcccaaa gtgc 44
<210> 62
   <211> 43
   <212> DNA
   <213> synthetic
<400> 62
   gcaacactgt gacgtactgg aggagccacc acacccagcc agg 43
<210> 63
   <211> 48
   <212> DNA
   <213> synthetic
<400> 63
   gcaacactgt gacgtactgg aggaataact gttttctccc cccaaaac 48
<210> 64
   <211> 48
   <212> DNA
   <213> synthetic
<400> 64
   gcaacactgt gacgtactgg aggtgtttta caaaaatggg gccatacc 48
<210> 65
   <211> 51
   <212> DNA
   <213> synthetic
<400> 65
   gcaacactgt gacgtactgg aggacttaag caaattcttt cataaaaagg g 51
<210> 66
   <211> 48
   <212> DNA
   <213> synthetic
<400> 66
   gcaacactgt gacgtactgg aggctttcaa ttgttgtacc aactctcc 48
<210> 67
   <211> 46
   <212> DNA
   <213> synthetic
<400> 67
   gcaacactgt gacgtactgg aggacctcct gcatctctcc ttttgc 46
<210> 68
   <211> 50
   <212> DNA
   <213> synthetic
<400> 68
   gcaacactgt gacgtactgg aggaaataaa gttttgagaa ccataagtgg 50
<210> 69
   <211> 45
   <212> DNA
   <213> synthetic
<400> 69
   gcaacactgt gacgtactgg aggcaccatc acagctcact gcagc 45
<210> 70
   <211> 47
   <212> DNA
   <213> synthetic
<400> 70
   gcaacactgt gacgtactgg aggaacctct ttgagaatcg gatagcc 47
<210> 71
   <211> 50
   <212> DNA
   <213> synthetic
<400> 71
   gcaacactgt gacgtactgg aggaaataaa gtacatacct ccaattttgc 50
<210> 72
   <211> 48
   <212> DNA
   <213> synthetic
<400> 72
   gcaacactgt gacgtactgg agggacacat tcctatgggt ttaattcc 48
<210> 73
   <211> 49
   <212> DNA
   <213> synthetic
<400> 73
   gcaacactgt gacgtactgg aggtgtaaaa tatggtttca gaagggagg 49
<210> 74
   <211> 45
   <212> DNA
   <213> synthetic
<400> 74
   gcaacactgt gacgtactgg agggcaggtg gataacgagg tcagg 45
<210> 75
   <211> 48
   <212> DNA
   <213> synthetic
<400> 75
   gcaacactgt gacgtactgg aggccagcca agaatttcaa agattagc 48
<210> 76
   <211> 46
   <212> DNA
   <213> synthetic
<400> 76
   gcaacactgt gacgtactgg agggaaggga gatgacaaag ggaacg 46
<210> 77
   <211> 46
   <212> DNA
   <213> synthetic
<400> 77
   gcaacactgt gacgtactgg agggcagaag aactgcttga acctgg 46
<210> 78
   <211> 45
   <212> DNA
   <213> synthetic
<400> 78
   gcaacactgt gacgtactgg agggtggtcc cagctactcg agagg 45
<210> 79
   <211> 48
   <212> DNA
   <213> synthetic
<400> 79
   gcaacactgt gacgtactgg aggccctcag caaaactaac tgaaaagg 48
<210> 80
   <211> 49
   <212> DNA
   <213> synthetic
<400> 80
   gcaacactgt gacgtactgg aggtagaaac caagatatct agaattccc 49
<210> 81
   <211> 45
   <212> DNA
   <213> synthetic
<400> 81
   gcaacactgt gacgtactgg aggccacgcc cggcggaata aatgc 45
<210> 82
   <211> 49
   <212> DNA
   <213> synthetic
<400> 82
   gcaacactgt gacgtactgg aggacaaaaa aagaggcaaa aactgagag 49
<210> 83
   <211> 44
   <212> DNA
   <213> synthetic
<400> 83
   gcaacactgt gacgtactgg aggctgggcg cagtggctca tgcc 44
<210> 84
   <211> 45
   <212> DNA
   <213> synthetic
<400> 84
   gcaacactgt gacgtactgg aggtggctgt gaggctgaga actgc 45
<210> 85
   <211> 45
   <212> DNA
   <213> synthetic
<400> 85
   gcaacactgt gacgtactgg aggctgggcg acagagtgag actcc 45
<210> 86
   <211> 44
   <212> DNA
   <213> synthetic
<400> 86
   gcaacactgt gacgtactgg aggaagtctg gctgggcgca gtgg 44
<210> 87
   <211> 47
   <212> DNA
   <213> synthetic
<400> 87
   gcaacactgt gacgtactgg aggaatggac aaaagaggtg aactggc 47
<210> 88
   <211> 47
   <212> DNA
   <213> synthetic
<400> 88
   gcaacactgt gacgtactgg agggatagag tgaaaacgca caatggc 47
<210> 89
   <211> 49
   <212> DNA
   <213> synthetic
<400> 89
   gcaacactgt gacgtactgg aggaattaaa cagctaggtc aatatgagg 49
<210> 90
   <211> 46
   <212> DNA
   <213> synthetic
<400> 90
   gcaacactgt gacgtactgg aggggtctcc actatcaagg gacaag 46
<210> 91
   <211> 47
   <212> DNA
   <213> synthetic
<400> 91
   gcaacactgt gacgtactgg aggaagcagc tgttagtcat ttccagg 47
<210> 92
   <211> 46
   <212> DNA
   <213> synthetic
<400> 92
   gcaacactgt gacgtactgg aggaggcatc ctcagattat ggctcc 46
<210> 93
   <211> 46
   <212> DNA
   <213> synthetic
<400> 93
   gcaacactgt gacgtactgg aggcctgagt aacactgaga ccctgc 46
<210> 94
   <211> 47
   <212> DNA
   <213> synthetic
<400> 94
   gcaacactgt gacgtactgg aggaacactc aagctgtcaa gagacac 47
<210> 95
   <211> 44
   <212> DNA
   <213> synthetic
<400> 95
   gcaacactgt gacgtactgg aggattcagg ccaggcgcag tggc 44
<210> 96
   <211> 47
   <212> DNA
   <213> synthetic
<400> 96
   gcaacactgt gacgtactgg aggtaaatcg taaaactgcc acaaagc 47
<210> 97
   <211> 47
   <212> DNA
   <213> synthetic
<400> 97
   gcaacactgt gacgtactgg aggcagagga gtaggagaag gaaaagg 47
<210> 98
   <211> 48
   <212> DNA
   <213> synthetic
<400> 98
   gcaacactgt gacgtactgg aggggtagct atctaccaag tagaatcc 48
<210> 99
   <211> 48
   <212> DNA
   <213> synthetic
<400> 99
   gcaacactgt gacgtactgg aggatcagat tggaaaaagt cccaaagc 48
<210> 100
   <211> 46
   <212> DNA
   <213> synthetic
<400> 100
   gcaacactgt gacgtactgg aggctcctga aaagcaccta ctcagc 46
<210> 101
   <211> 46
   <212> DNA
   <213> synthetic
<400> 101
   gcaacactgt gacgtactgg aggctcctta aacctgaggt actggg 46
<210> 102
   <211> 48
   <212> DNA
   <213> synthetic
<400> 102
   gcaacactgt gacgtactgg aggttttctc ctaatagacc accattcc 48
<210> 103
   <211> 48
   <212> DNA
   <213> synthetic
<400> 103
   gcaacactgt gacgtactgg aggctgctgt attaccatca ctcatgtc 48
<210> 104
   <211> 46
   <212> DNA
   <213> synthetic
<400> 104
   gcaacactgt gacgtactgg aggctggcca acatagtgaa accacg 46
<210> 105
   <211> 50
   <212> DNA
   <213> synthetic
<400> 105
   gcaacactgt gacgtactgg aggatttgaa taggggttaa agtatcattg 50
<210> 106
   <211> 46
   <212> DNA
   <213> synthetic
<400> 106
   gcaacactgt gacgtactgg aggcacttca gtggaagttg gcatgc 46
<210> 107
   <211> 50
   <212> DNA
   <213> synthetic
<400> 107
   gcaacactgt gacgtactgg agggtttttc ttcgaagtga taaacatacg 50
<210> 108
   <211> 47
   <212> DNA
   <213> synthetic
<400> 108
   gcaacactgt gacgtactgg agggctcctt agtctatgta cctgtgg 47
<210> 109
   <211> 46
   <212> DNA
   <213> synthetic
<400> 109
   gcaacactgt gacgtactgg aggtactctg gcatggtaac tggtgc 46
<210> 110
   <211> 46
   <212> DNA
   <213> synthetic
<400> 110
   gcaacactgt gacgtactgg aggacaaagg actaggtctg tggagc 46
<210> 111
   <211> 51
   <212> DNA
   <213> synthetic
<400> 111
   gcaacactgt gacgtactgg aggccaagtt taccaaatta ccaaagttac c 51
<210> 112
   <211> 45
   <212> DNA
   <213> synthetic
<400> 112
   gcaacactgt gacgtactgg aggtgagccg atatcacgcc actgc 45
<210> 113
   <211> 47
   <212> DNA
   <213> synthetic
<400> 113
   gcaacactgt gacgtactgg aggtcccaat aaaggttttg gcccagg 47
<210> 114
   <211> 47
   <212> DNA
   <213> synthetic
<400> 114
   gcaacactgt gacgtactgg aggctgggta gcaaattagg gaacagg 47
<210> 115
   <211> 48
   <212> DNA
   <213> synthetic
<400> 115
   gcaacactgt gacgtactgg aggctggcca gaaaagacag ttttatcc 48
<210> 116
   <211> 46
   <212> DNA
   <213> synthetic
<400> 116
   gcaacactgt gacgtactgg aggggttccc aggaagggat aacacc 46
<210> 117
   <211> 46
   <212> DNA
   <213> synthetic
<400> 117
   gcaacactgt gacgtactgg aggtcactcc aggaggttcc atttcc 46
<210> 118
   <211> 47
   <212> DNA
   <213> synthetic
<400> 118
   gcaacactgt gacgtactgg aggaggcttg gaaataagca gcagtgg 47
<210> 119
   <211> 49
   <212> DNA
   <213> synthetic
<400> 119
   gcaacactgt gacgtactgg aggattcata caatggaata ctactcagc 49
<210> 120
   <211> 46
   <212> DNA
   <213> synthetic
<400> 120
   gcaacactgt gacgtactgg aggtaagtga tcctcccacc tcaacc 46
<210> 121
   <211> 46
   <212> DNA
   <213> synthetic
<400> 121
   gcaacactgt gacgtactgg aggtataaga ggaagactgg ggctgg 46
<210> 122
   <211> 48
   <212> DNA
   <213> synthetic
<400> 122
   gcaacactgt gacgtactgg aggtcatact tatgcaggtt ataggagg 48
<210> 123
   <211> 45
   <212> DNA
   <213> synthetic
<400> 123
   gcaacactgt gacgtactgg aggcaagatc acgccactgc actcc 45
<210> 124
   <211> 49
   <212> DNA
   <213> synthetic
<400> 124
   gcaacactgt gacgtactgg aggaaaataa atagctggtg ctcaagatc 49
<210> 125
   <211> 46
   <212> DNA
   <213> synthetic
<400> 125
   gcaacactgt gacgtactgg aggcaccagc ctcattcaac agatgg 46
<210> 126
   <211> 45
   <212> DNA
   <213> synthetic
<400> 126
   gcaacactgt gacgtactgg aggcaatgca gcctcaacct cctgg 45
<210> 127
   <211> 46
   <212> DNA
   <213> synthetic
<400> 127
   gcaacactgt gacgtactgg agggttaggt caggtgctca tgtctg 46
<210> 128
   <211> 50
   <212> DNA
   <213> synthetic
<400> 128
   gcaacactgt gacgtactgg aggaagtttc aaaaggacat gtacaaaatg 50
<210> 129
   <211> 45
   <212> DNA
   <213> synthetic
<400> 129
   gcaacactgt gacgtactgg aggtcctgaa gaggctgcag cttcc 45
<210> 130
   <211> 46
   <212> DNA
   <213> synthetic
<400> 130
   gcaacactgt gacgtactgg aggctggtgc acattcccaa gtgtgc 46
<210> 131
   <211> 47
   <212> DNA
   <213> synthetic
<400> 131
   gcaacactgt gacgtactgg aggcatgttg gccatgttct tctgagg 47
<210> 132
   <211> 44
   <212> DNA
   <213> synthetic
<400> 132
   gcaacactgt gacgtactgg aggctcagcc tcccgagtag ctgg 44
<210> 133
   <211> 48
   <212> DNA
   <213> synthetic
<400> 133
   gcaacactgt gacgtactgg aggaaagaca tttaagagga gatgaggc 48
<210> 134
   <211> 45
   <212> DNA
   <213> synthetic
<400> 134
   gcaacactgt gacgtactgg aggtgctggg attacaggcg tgagc 45
<210> 135
   <211> 45
   <212> DNA
   <213> synthetic
<400> 135
   gcaacactgt gacgtactgg aggtgtgact tccatccgca gctcc 45
<210> 136
   <211> 47
   <212> DNA
   <213> synthetic
<400> 136
   gcaacactgt gacgtactgg agggacactt ttgtggagct ttcatgg 47
<210> 137
   <211> 45
   <212> DNA
   <213> synthetic
<400> 137
   gcaacactgt gacgtactgg aggcatgtga gggggcacgt cttgc 45
<210> 138
   <211> 48
   <212> DNA
   <213> synthetic
<400> 138
   gcaacactgt gacgtactgg aggtcttctc tatgagaaaa gtggttgc 48
<210> 139
   <211> 46
   <212> DNA
   <213> synthetic
<400> 139
   gcaacactgt gacgtactgg aggtggcaaa atgctatcga gctgcc 46
<210> 140
   <211> 45
   <212> DNA
   <213> synthetic
<400> 140
   gcaacactgt gacgtactgg aggtatgaac acagccggcc tcagg 45
<210> 141
   <211> 46
   <212> DNA
   <213> synthetic
<400> 141
   gcaacactgt gacgtactgg agggaggttg cagtgagctg agatcg 46
<210> 142
   <211> 45
   <212> DNA
   <213> synthetic
<400> 142
   gcaacactgt gacgtactgg agggtcaagc acccagtccg atacc 45
<210> 143
   <211> 44
   <212> DNA
   <213> synthetic
<400> 143
   gcaacactgt gacgtactgg aggatctggg cttggtggcg cacg 44
<210> 144
   <211> 45
   <212> DNA
   <213> synthetic
<400> 144
   gcaacactgt gacgtactgg agggttaagc gggtcccaca tcagc 45
<210> 145
   <211> 47
   <212> DNA
   <213> synthetic
<400> 145
   gcaacactgt gacgtactgg aggcagccag tttcagtaga aagatgc 47
<210> 146
   <211> 46
   <212> DNA
   <213> synthetic
<400> 146
   gcaacactgt gacgtactgg agggacccaa gcataagggg actagc 46
<210> 147
   <211> 50
   <212> DNA
   <213> synthetic
<400> 147
   gcaacactgt gacgtactgg aggcccaaaa agtttacaag agaaattttc 50
<210> 148
   <211> 45
   <212> DNA
   <213> synthetic
<400> 148
   gcaacactgt gacgtactgg aggcgcctgt agtcccagct actcg 45
<210> 149
   <211> 46
   <212> DNA
   <213> synthetic
<400> 149
   gcaacactgt gacgtactgg aggcgcgtga tgcggaaaag aaatcc 46
<210> 150
   <211> 47
   <212> DNA
   <213> synthetic
<400> 150
   gcaacactgt gacgtactgg aggtctacta tgaaccctcc ttcagac 47
<210> 151
   <211> 46
   <212> DNA
   <213> synthetic
<400> 151
   gcaacactgt gacgtactgg agggtgctgg gattacaggt gtgagc 46
<210> 152
   <211> 46
   <212> DNA
   <213> synthetic
<400> 152
   gcaacactgt gacgtactgg aggttatcca aatgtcccag ggcagg 46
<210> 153
   <211> 44
   <212> DNA
   <213> synthetic
<400> 153
   gcaacactgt gacgtactgg aggctgccag cactgctcgc cagc 44
<210> 154
   <211> 45
   <212> DNA
   <213> synthetic
<400> 154
   gcaacactgt gacgtactgg agggctactg caggcagtgc cttcc 45
<210> 155
   <211> 45
   <212> DNA
   <213> synthetic
<400> 155
   gcaacactgt gacgtactgg aggcatccaa gcccaaggtg tcagg 45
<210> 156
   <211> 48
   <212> DNA
   <213> synthetic
<400> 156
   gcaacactgt gacgtactgg aggtgtttgc atgtaatttc aggaagcc 48
<210> 157
   <211> 47
   <212> DNA
   <213> synthetic
<400> 157
   gcaacactgt gacgtactgg agggatccgt cactgttaac actcagg 47
<210> 158
   <211> 46
   <212> DNA
   <213> synthetic
<400> 158
   gcaacactgt gacgtactgg aggctcacag tcacaagctc ctgagc 46
<210> 159
   <211> 45
   <212> DNA
   <213> synthetic
<400> 159
   gcaacactgt gacgtactgg agggagatga tgctggggtc acagg 45
<210> 160
   <211> 46
   <212> DNA
   <213> synthetic
<400> 160
   gcaacactgt gacgtactgg aggttagaag aatgggatcg caaagg 46
<210> 161
   <211> 47
   <212> DNA
   <213> synthetic
<400> 161
   gcaacactgt gacgtactgg aggcggtatt caaatatgag gtcaggc 47
<210> 162
   <211> 46
   <212> DNA
   <213> synthetic
<400> 162
   gcaacactgt gacgtactgg agggtaaatc ctgctgccag tcttcc 46
<210> 163
   <211> 45
   <212> > DNA
   <213> synthetic
<400> 163
   gcaacactgt gacgtactgg aggacagggt cagacagagc cttgg 45
<210> 164
   <211> 50
   <212> DNA
   <213> synthetic
<400> 164
   gcaacactgt gacgtactgg aggagttatt gatctaacta tacaacaagc 50
<210> 165
   <211> 44
   <212> DNA
   <213> synthetic
<400> 165
   gcaacactgt gacgtactgg aggaaagact aggggccggg gacg 44
<210> 166
   <211> 49
   <212> DNA
   <213> synthetic
<400> 166
   gcaacactgt gacgtactgg aggctggtag aaataaagac aacaaagcc 49
<210> 167
   <211> 50
   <212> DNA
   <213> synthetic
<400> 167
   gcaacactgt gacgtactgg agggtgccaa gtaattaaaa gtttgaaacc 50
<210> 168
   <211> 45
   <212> DNA
   <213> synthetic
<400> 168
   gcaacactgt gacgtactgg aggggctttt gaagggagca ccacc 45
<210> *169
   <211> 50
   <212> DNA
   <213> synthetic
<400> 169
   gcaacactgt gacgtactgg agggaaggat aaatacctat gatactttcc 50
<210> 170
   <211> 47
   <212> DNA
   <213> synthetic
<400> 170
   gcaacactgt gacgtactgg aggggcaggg aaatactgtg cttcaag 47
<210> 171
   <211> 47
   <212> DNA
   <213> synthetic
<400> 171
   gcaacactgt gacgtactgg agggtggtga aattccacct cagtacc 47
<210> 172
   <211> 46
   <212> DNA
   <213> synthetic
<400> 172
   gcaacactgt gacgtactgg aggtcccaaa gtgctgggat tacagg 46
<210> 173
   <211> 48
   <212> DNA
   <213> synthetic
<400> 173
   gcaacactgt gacgtactgg agggaaatta gcaaacaatg ccaagacg 48
<210> 174
   <211> 46
   <212> DNA
   <213> synthetic
<400> 174
   gcaacactgt gacgtactgg aggtaagtat tggaccggga aggagg 46
<210> 175
   <211> 48
   <212> DNA
   <213> synthetic
<400> 175
   gcaacactgt gacgtactgg aggctatcat tttgctcaaa gtgtagcc 48
<210> 176
   <211> 47
   <212> DNA
   <213> synthetic
<400> 176
   gcaacactgt gacgtactgg aggatttcac aaactacaga ggccagg 47
<210> 177
   <211> 47
   <212> DNA
   <213> synthetic
<400> 177
   gcaacactgt gacgtactgg aggtagactt ctgtctctct atgctgc 47
<210> 178
   <211> 46
   <212> DNA
   <213> synthetic
<400> 178
   gcaacactgt gacgtactgg aggtgagtga gctgccatgt gatacc 46
<210> 179
   <211> 45
   <212> DNA
   <213> synthetic
<400> 179
   gcaacactgt gacgtactgg aggacttcac accagcctgt ccacc 45
<210> 180
   <211> 47
   <212> DNA
   <213> synthetic
<400> 180
   gcaacactgt gacgtactgg aggtaactca tatcctcaga gagaccc 47
<210> 181
   <211> 45
   <212> DNA
   <213> synthetic
<400> 181
   gcaacactgt gacgtactgg aggagaggtt cctcgattcc cctgc 45
<210> 182
   <211> 46
   <212> DNA
   <213> synthetic
<400> 182
   gcaacactgt gacgtactgg agggtgtcag cgtcccaaca caaagc 46
<210> 183
   <211> 46
   <212> DNA
   <213> synthetic
<400> 183
   gcaacactgt gacgtactgg agggaaagtg gatgggcaag cattgc 46
<210> 184
   <211> 45
   <212> DNA
   <213> synthetic
<400> 184
   gcaacactgt gacgtactgg agggtgatca cctcacagct gcagg 45
<210> 185
   <211> 48
   <212> DNA
   <213> synthetic
<400> 185
   gcaacactgt gacgtactgg agggtttgtt tagtcaaggc atttcacc 48
<210> 186
   <211> 45
   <212> DNA
   <213> synthetic
<400> 186
   gcaacactgt gacgtactgg aggcctcagc ctccagagta gctgg 45
<210> 187
   <211> 47
   <212> DNA
   <213> synthetic
<400> 187
   gcaacactgt gacgtactgg aggtaaaaga aaactcctcc ttcctgg 47
<210> 188
   <211> 49
   <212> DNA
   <213> synthetic
<400> 188
   gcaacactgt gacgtactgg aggaatgtgc tatgtcttta aatccatgg 49
<210> 189
   <211> 49
   <212> DNA
   <213> synthetic
<400> 189
   gcaacactgt gacgtactgg aggagctggc aaatctggta atataaaag 49
<210> 190
   <211> 45
   <212> DNA
   <213> synthetic
<400> 190
   gcaacactgt gacgtactgg agggcttgaa cctggaaggt ggagg 45
<210> 191
   <211> 46
   <212> DNA
   <213> synthetic
<400> 191
   gcaacactgt gacgtactgg agggcaggca tgctaagacc ttcagc 46
<210> 192
   <211> 47
   <212> DNA
   <213> synthetic
<400> 192
   gcaacactgt gacgtactgg aggcagctcc atgaataact ccacagg 47
<210> 193
   <211> 45
   <212> DNA
   <213> synthetic
<400> 193
   gcaacactgt gacgtactgg agggcttgaa cccaggaggc agagg 45
<210> 194
   <211> 46
   <212> DNA
   <213> synthetic
<400> 194
   gcaacactgt gacgtactgg aggatcgaag atgccactgc aagagg 46
<210> 195
   <211> 46
   <212> DNA
   <213> synthetic
<400> 195
   gcaacactgt gacgtactgg aggccaacca cacttcaggg gatacc 46
<210> 196
   <211> 46
   <212> DNA
   <213> synthetic
<400> 196
   gcaacactgt gacgtactgg aggcacgcca gtccactgat actcac 46
<210> 197
   <211> 45
   <212> DNA
   <213> synthetic
<400> 197
   gcaacactgt gacgtactgg agggggtttc accatgttgg ccagg 45
<210> 198
   <211> 45
   <212> DNA
   <213> synthetic
<400> 198
   gcaacactgt gacgtactgg aggcccaaca aaggctctgg cctgg 45
<210> 199
   <211> 46
   <212> DNA
   <213> synthetic
<400> 199
   gcaacactgt gacgtactgg aggatgacag cagaggagct tcatcc 46
<210> 200
   <211> 46
   <212> DNA
   <213> synthetic
<400> 200
   gcaacactgt gacgtactgg agggcaggct acgagtaaaa ggatgg 46
<210> 201
   <211> 46
   <212> DNA
   <213> synthetic
<400> 201
   gcaacactgt gacgtactgg aggcgggtaa aatcttgcct ccttcc 46
<210> 202
   <211> 48
   <212> DNA
   <213> synthetic
<400> 202
   gcaacactgt gacgtactgg aggaaactta aaccaatggt ggatgtgg 48
<210> 203
   <211> 46
   <212> DNA
   <213> synthetic
<400> 203
   gcaacactgt gacgtactgg aggagagact gaggaactgt tccagc 46
<210> 204
   <211> 47
   <212> DNA
   <213> synthetic
<400> 204
   gcaacactgt gacgtactgg agggaaacgg tcttggatca ctgatcc 47
<210> 205
   <211> 47
   <212> DNA
   <213> synthetic
<400> 205
   gcaacactgt gacgtactgg aggtgcgcat gatatcttgt ttcaggg 47
<210> 206
   <211> 46
   <212> DNA
   <213> synthetic
<400> 206
   gcaacactgt gacgtactgg aggggcctcc gtttaaactg ttgtgc 46
<210> 207
   <211> 45
   <212> DNA
   <213> synthetic
<400> 207
   gcaacactgt gacgtactgg agggaatgct ggcccgacac agtgg 45
<210> 208
   <211> 47
   <212> DNA
   <213> synthetic
<400> 208
   gcaacactgt gacgtactgg aggtcttggt atagaaaagc cagctgg 47
<210> 209
   <211> 45
   <212> DNA
   <213> synthetic
<400> 209
   gcaacactgt gacgtactgg agggcaaaag cccaagagcc cctgg 45
<210> 210
   <211> 46
   <212> DNA
   <213> synthetic
<400> 210
   gcaacactgt gacgtactgg aggttctccc aaaatgagcc ccaagg 46
<210> 211
   <211> 47
   <212> DNA
   <213> synthetic
<400> 211
   gcaacactgt gacgtactgg agggtggtga cgtaaacaaa aggtacc 47
<210> 212
   <211> 49
   <212> DNA
   <213> synthetic
<400> 212
   gcaacactgt gacgtactgg agggcaaatt ccatgtgaat cttattggc 49
<210> 213
   <211> 46
   <212> DNA
   <213> synthetic
<400> 213
   gcaacactgt gacgtactgg aggcctgatc tatggaacag tggtgg 46
<210> 214
   <211> 48
   <212> DNA
   <213> synthetic
<400> 214
   gcaacactgt gacgtactgg agggttacaa acgttgcagt ttgcaacg 48
<210> 215
   <211> 46
   <212> DNA
   <213> synthetic
<400> 215
   gcaacactgt gacgtactgg agggaacccc gtcaacagtg atcacc 46
<210> 216
   <211> 45
   <212> DNA
   <213> synthetic
<400> 216
   gcaacactgt gacgtactgg aggacaggac ctcaaggcaa ggagc 45
<210> 217
   <211> 50
   <212> DNA
   <213> synthetic
<400> 217
   gcaacactgt gacgtactgg aggcatacct aaaatagaaa tgtctatccc 50
<210> 218
   <211> 50
   <212> DNA
   <213> synthetic
<400> 218
   gcaacactgt gacgtactgg agggagttgc atatatgttt tataaatccc 50
<210> 219
   <211> 47
   <212> DNA
   <213> synthetic
<400> 219
   gcaacactgt gacgtactgg aggtgagccc acatccataa agttagc 47
<210> 220
   <211> 44
   <212> DNA
   <213> synthetic
<400> 220
   gcaacactgt gacgtactgg aggaccgcaa cctttgccgc ctgg 44
<210> 221
   <211> 49
   <212> DNA
   <213> synthetic
<400> 221
   gcaacactgt gacgtactgg aggtaaatat tttgtatgga gtcaccacc 49
<210> 222
   <211> 48
   <212> DNA
   <213> synthetic
<400> 222
   gcaacactgt gacgtactgg aggaaagcca ggagaaaaag ttatgagg 48
<210> 223
   <211> 46
   <212> DNA
   <213> synthetic
<400> 223
   gcaacactgt gacgtactgg aggtcccaaa gtcccaggat tacagg 46
<210> 224
   <211> 46
   <212> DNA
   <213> synthetic
<400> 224
   gcaacactgt gacgtactgg aggtcactat ggagcatctc cgatgg 46
<210> 225
   <211> 45
   <212> DNA
   <213> synthetic
<400> 225
   gcaacactgt gacgtactgg aggagttccc tggaagtctc cgagg 45
<210> 226
   <211> 47
   <212> DNA
   <213> synthetic
<400> 226
   gcaacactgt gacgtactgg aggaaaataa tcacccagcc cacatcc 47
<210> 227
   <211> 48
   <212> DNA
   <213> synthetic
<400> 227
   gcaacactgt gacgtactgg aggacaaaac tacagacaca gaaagtgg 48
<210> 228
   <211> 45
   <212> DNA
   <213> synthetic
<400> 228
   gcaacactgt gacgtactgg aggtttggga ggctgaggta ggtgg 45
<210> 229
   <211> 48
   <212> DNA
   <213> synthetic
<400> 229
   gcaacactgt gacgtactgg aggaaagaca gtgaaacatc tataaggg 48
<210> 230
   <211> 45
   <212> DNA
   <213> synthetic
<400> 230
   gcaacactgt gacgtactgg aggcattttg ggagaccagg gcagg 45
<210> 231
   <211> 46
   <212> DNA
   <213> synthetic
<400> 231
   gcaacactgt gacgtactgg agggcatggg acagacacaa agcagc 46
<210> 232
   <211> 46
   <212> DNA
   <213> synthetic
<400> 232
   gcaacactgt gacgtactgg agggaataac aaagagagcc ggctgg 46
<210> 233
   <211> 52
   <212> DNA
   <213> synthetic
<400> 233
   gcaacactgt gacgtactgg aggtaaacct tttattgaaa attgtcaaat gg 52
<210> 234
   <211> 44
   <212> DNA
   <213> synthetic
<400> 234
   gcaacactgt gacgtactgg aggcgcctca gcctcccaaa gtgc 44
<210> 235
   <211> 49
   <212> DNA
   <213> synthetic
<400> 235
   gcaacactgt gacgtactgg aggtacatta gttttatagg tccagtagg 49
<210> 236
   <211> 50
   <212> DNA
   <213> synthetic
<400> 236
   gcaacactgt gacgtactgg agggaaggtt tattcatatt aaaatgtgcc 50
<210> 237
   <211> 46
   <212> DNA
   <213> synthetic
<400> 237
   gcaacactgt gacgtactgg aggctggctt ctgtggtttg agttgg 46
<210> 238
   <211> 46
   <212> DNA
   <213> synthetic
<400> 238
   gcaacactgt gacgtactgg aggacagacc tacctcctaa ggatgg 46
<210> 239
   <211> 48
   <212> DNA
   <213> synthetic
<400> 239
   gcaacactgt gacgtactgg agggctagct tttgtgtgta agaatggg 48
<210> 240
   <211> 47
   <212> DNA
   <213> synthetic
<400> 240
   gcaacactgt gacgtactgg aggggcctac tcacacaata gaatacc 47
<210> 241
   <211> 45
   <212> DNA
   <213> synthetic
<400> 241
   gcaacactgt gacgtactgg agggcaccat tgcactccag cctgg 45
<210> 242
   <211> 49
   <212> DNA
   <213> synthetic
<400> 242
   gcaacactgt gacgtactgg agggaaatta ggataaaggt tgtcacagc 49
<210> 243
   <211> 48
   <212> DNA
   <213> synthetic
<400> 243
   gcaacactgt gacgtactgg aggcagaagt gttcaaggtg aaactgtc 48
<210> 244
   <211> 49
   <212> DNA
   <213> synthetic
<400> 244
   gcaacactgt gacgtactgg aggctgaatc atgaaatgtt ctactctgc 49
<210> 245
   <211> 46
   <212> DNA
   <213> synthetic
<400> 245
   gcaacactgt gacgtactgg aggtgtcaac ttgactgggc catacg 46
<210> 246
   <211> 47
   <212> DNA
   <213> synthetic
<400> 246
   gcaacactgt gacgtactgg aggctcccgt atagttggga ttatagg 47
<210> 247
   <211> 48
   <212> DNA
   <213> synthetic
<400> 247
   gcaacactgt gacgtactgg agggcttgga gttccttgaa attcttgg 48
<210> 248
   <211> 46
   <212> DNA
   <213> synthetic
<400> 248
   gcaacactgt gacgtactgg aggcctggtg gctccagttt tctacc 46
<210> 249
   <211> 46
   <212> DNA
   <213> synthetic
<400> 249
   gcaacactgt gacgtactgg aggaactcct gacctcatga tccacc 46
<210> > 250
   <211> 45
   <212> DNA
   <213> synthetic
<400> 250
   gcaacactgt gacgtactgg agggctggga ttacaggcat gagcc 45
<210> 251
   <211> 48
   <212> DNA
   <213> synthetic
<400> 251
   gcaacactgt gacgtactgg aggttctcct ttatccttgg tgacattc 48
<210> 252
   <211> 46
   <212> DNA
   <213> synthetic
<400> 252
   gcaacactgt gacgtactgg aggtcccaaa gtgctgggat tacagg 46
<210> 253
   <211> 46
   <212> DNA
   <213> synthetic
<400> 253
   gcaacactgt gacgtactgg agggtcataa gtcagggacc atctgc 46
<210> 254
   <211> 48
   <212> DNA
   <213> synthetic
<400> 254
   gcaacactgt gacgtactgg aggctgtttc attgatttcc agactggc 48
<210> 255
   <211> 45
   <212> DNA
   <213> synthetic
<400> 255
   gcaacactgt gacgtactgg agggcaatct cggctcactg caagc 45
<210> 256
   <211> 48
   <212> DNA
   <213> synthetic
<400> 256
   gcaacactgt gacgtactgg agggaagaag tgactatatc agatctgg 48
<210> 257
   <211> 45
   <212> DNA
   <213> synthetic
<400> 257
   gcaacactgt gacgtactgg aggttcacca tgttggccag gctgg 45
<210> 258
   <211> 47
   <212> DNA
   <213> synthetic
<400> 258
   gcaacactgt gacgtactgg aggcatcact gaagatgaca actgagc 47
<210> 259
   <211> 44
   <212> DNA
   <213> synthetic
<400> 259
   gcaacactgt gacgtactgg agggtccagc ctgggcgata gage 44
<210> 260
   <211> 47
   <212> DNA
   <213> synthetic
<400> 260
   gcaacactgt gacgtactgg agggaggaaa gtctttgaag aggaacc 47
<210> 261
   <211> 46
   <212> DNA
   <213> synthetic
<400> 261
   gcaacactgt gacgtactgg aggggtacac tcaccagcag ttttgc 46
<210> 262
   <211> 48
   <212> DNA
   <213> synthetic
<400> 262
   gcaacactgt gacgtactgg agggagcaac tggtgtgaat acatatgg 48
<210> 263
   <211> 46
   <212> DNA
   <213> synthetic
<400> 263
   gcaacactgt gacgtactgg aggcaatacc tggcaccaca tacacc 46
<210> 264
   <211> 45
   <212> DNA
   <213> synthetic
<400> 264
   gcaacactgt gacgtactgg agggggacta caggcatgtg ccacc 45
<210> 265
   <211> 45
   <212> DNA
   <213> synthetic
<400> 265
   gcaacactgt gacgtactgg aggcggtggc tcacgcgtgt aatcc 45
<210> 266
   <211> 49
   <212> DNA
   <213> synthetic
<400> 266
   gcaacactgt gacgtactgg aggcaactgt taaatctctc atggaaacc 49
<210> 267
   <211> 46
   <212> DNA
   <213> synthetic
<400> 267
   gcaacactgt gacgtactgg agggacaaag gattagaaat gcaccc 46
<210> 268
   <211> 49
   <212> DNA
   <213> synthetic
<400> 268
   gcaacactgt gacgtactgg aggggaaatg ttctaaaact ggattgtgg 49
<210> 269
   <211> 48
   <212> DNA
   <213> synthetic
<400> 269
   gcaacactgt gacgtactgg aggaataata atagccaggt gtggtagc 48
<210> 270
   <211> 46
   <212> DNA
   <213> synthetic
<400> 270
   gcaacactgt gacgtactgg aggctggaac actcacacat tgctgg 46
<210> 271
   <211> 45
   <212> DNA
   <213> synthetic
<400> 271
   gcaacactgt gacgtactgg aggctgggtg acagagcgag actcc 45
<210> 272
   <211> 48
   <212> DNA
   <213> synthetic
<400> 272
   gcaacactgt gacgtactgg aggcccaaat catccccgtg aaacatgc 48
<210> 273
   <211> 45
   <212> DNA
   <213> synthetic
<400> 273
   gcaacactgt gacgtactgg agggaccctg caatcccaac actgg 45
<210> 274
   <211> 46
   <212> DNA
   <213> synthetic
<400> 274
   gcaacactgt gacgtactgg aggctctcag gccttcaaac tacacc 46
<210> 275
   <211> 45
   <212> DNA
   <213> synthetic
<400> 275
   gcaacactgt gacgtactgg aggcaggaaa gggctcgctc agtgg 45
<210> 276
   <211> 46
   <212> DNA
   <213> synthetic
<400> 276
   gcaacactgt gacgtactgg aggatctgca aaagcagcag agcagg 46
<210> 277
   <211> 48
   <212> DNA
   <213> synthetic
<400> 277
   gcaacactgt gacgtactgg agggtaccca tgacagacaa gttttagg 48
<210> 278
   <211> 47
   <212> DNA
   <213> synthetic
<400> 278
   gcaacactgt gacgtactgg aggcttatcc cctactgtct cctttgg 47
<210> 279
   <211> 45
   <212> DNA
   <213> synthetic
<400> 279
   gcaacactgt gacgtactgg aggggatggt ctcgatctcc tgacc 45
<210> 280
   <211> 47
   <212> DNA
   <213> synthetic
<400> 280
   gcaacactgt gacgtactgg aggaggttag agaccttcct ctaatgc 47
<210> 281
   <211> 45
   <212> DNA
   <213> synthetic
<400> 281
   gcaacactgt gacgtactgg aggagctggg attacaggtg cctgc 45
<210> 282
   <211> 44
   <212> DNA
   <213> synthetic
<400> 282
   gcaacactgt gacgtactgg agggctgagg caggttgggg ctgc 44
<210> 283
   <211> 48
   <212> DNA
   <213> synthetic
<400> 283
   gcaacactgt gacgtactgg aggacattta acgtctccta acttctcc 48
<210> 284
   <211> 46
   <212> DNA
   <213> synthetic
<400> 284
   gcaacactgt gacgtactgg agggtgctgc gattacaggt gtgagc 46
<210> 285
   <211> 49
   <212> DNA
   <213> synthetic
<400> 285
   gcaacactgt gacgtactgg aggtatgaca gcagtattat actatcacc 49
<210> 286
   <211> 46
   <212> DNA
   <213> synthetic
<400> 286
   gcaacactgt gacgtactgg aggctgggga ccaaatctga actgcc 46
<210> 287
   <211> 49
   <212> DNA
   <213> synthetic
<400> 287
   gcaacactgt gacgtactgg agggtagcta ttgttatttc caaaagagg 49
<210> 288
   <211> 44
   <212> DNA
   <213> synthetic
<400> 288
   gcaacactgt gacgtactgg agggcttggg accccaggac aagg 44
<210> 289
   <211> 45
   <212> DNA
   <213> synthetic
<400> 289
   gcaacactgt gacgtactgg aggcctggcc aacatgggga aatcc 45
<210> 290
   <211> 46
   <212> DNA
   <213> synthetic
<400> 290
   gcaacactgt gacgtactgg aggaattgct tgaacctggg aggtgg 46
<210> 291
   <211> 47
   <212> DNA
   <213> synthetic
<400> 291
   gcaacactgt gacgtactgg agggcctaag acccaaaagc tattagc 47
<210> 292
   <211> 50
   <212> DNA
   <213> synthetic
<400> 292
   gcaacactgt gacgtactgg aggcatatta aagggccata ttcaaattgg 50
<210> 293
   <211> 48
   <212> DNA
   <213> synthetic
<400> 293
   gcaacactgt gacgtactgg aggggatgta accagtgtat atcacagg 48
<210> 294
   <211> 48
   <212> DNA
   <213 > synthetic
<400> 294
   gcaacactgt gacgtactgg aggggaagtt tagtccacat cttctagc 48
<210> 295
   <211> 45
   <212> DNA
   <213> synthetic
<400> 295
   gcaacactgt gacgtactgg agggcaccca caggacaacc acacg 45
<210> 296
   <211> 45
   <212> DNA
   <213> synthetic
<400> 296
   gcaacactgt gacgtactgg agggggacgc gcctgttaac aaagg 45
<210> 297
   <211> 42
   <212> DNA
   <213> synthetic
<400> 297
   gcaacactgt gacgtactgg agggggctgg gggccacgct cc 42
<210> 298
   <211> 45
   <212> DNA
   <213> synthetic
<400> 298
   gcaacactgt gacgtactgg aggcgcaaaa gtgaagccct cctgg 45
<210> 299
   <211> 49
   <212> DNA
   <213> synthetic
<400> 299
   gcaacactgt gacgtactgg agggaaatcc tacttgatct aaagtgagc 49
<210> 300
   <211> 49
   <212> DNA
   <213> synthetic
<400> 300
   gcaacactgt gacgtactgg aggtttgagc aacttggaaa aaataagcg 49
<210> 301
   <211> 48
   <212> DNA
   <213> synthetic
<400> 301
   gcaacactgt gacgtactgg aggttcccaa aagacaaata gcacttcc 48
<210> > 302
   <211> 49
   <212> DNA
   <213> synthetic
<400> 302
   gcaacactgt gacgtactgg aggccatttt gaaaatcaca gtgaattcc 49
<210> 303
   <211> 49
   <212> DNA
   <213> synthetic
<400> 303
   gcaacactgt gacgtactgg agggaaaaga aaaccctgaa ttcaaaagg 49
<210> 304
   <211> 49
   <212> DNA
   <213> synthetic
<400> 304
   gcaacactgt gacgtactgg aggtgctgaa aagaagcatt taaaagtgg 49
<210> 305
   <211> 47
   <212> DNA
   <213> synthetic
<400> 305
   gcaacactgt gacgtactgg aggctcttac cagtttcaga gctttcc 47
<210> 306
   <211> 48
   <212> DNA
   <213> synthetic
<400> 306
   gcaacactgt gacgtactgg aggttttcag ccaaaaatca aggacagg 48
<210> 307
   <211> 46
   <212> DNA
   <213> synthetic
<400> 307
   gcaacactgt gacgtactgg aggcttgagc ccaggagttt gagacc 46
<210> 308
   <211> 46
   <212> DNA
   <213> synthetic
<400> 308
   gcaacactgt gacgtactgg aggcgcctgt agtaccctct actagg 46
<210> 309
   <211> 49
   <212> DNA
   <213> synthetic
<400> 309
   gcaacactgt gacgtactgg aggggtaaag aaagaaggat ttgaaaacc 49
<210> 310
   <211> 50
   <212> DNA
   <213> synthetic
<400> 310
   gcaacactgt gacgtactgg aggtaagagt aatgaggtta aagtttatgc 50
<210> 311
   <211> 49
   <212> DNA
   <213> synthetic
<400> 311
   gcaacactgt gacgtactgg aggcattttt attgtcacag gccatttgc 49
<210> 312
   <211> 46
   <212> DNA
   <213> synthetic
<400> 312
   gcaacactgt gacgtactgg agggccacgc cttctcttct gccacc 46
<210> 313
   <211> 45
   <212> DNA
   <213> synthetic
<400> 313
   gcaacactgt gacgtactgg aggtgcctct cctgactgca ctgtg 45
<210> 314
   <211> 45
   <212> DNA
   <213> synthetic
<400> 314
   gcaacactgt gacgtactgg aggccatgct ctaccacgcc cttgg 45
<210> 315
   <211> 45
   <212> DNA
   <213> synthetic
<400> 315
   gcaacactgt gacgtactgg aggcattcag gctggagtgc ggtgg 45
<210> 316
   <211> 50
   <212> DNA
   <213> synthetic
<400> 316
   gcaacactgt gacgtactgg aggcttaaaa attgtctggc taagacattg 50
<210> 317
   <211> 45
   <212> DNA
   <213> synthetic
<400> 317
   gcaacactgt gacgtactgg aggttgctct tgttgcccgg gttgg 45
<210> 318
   <211> 49
   <212> DNA
   <213> synthetic
<400> 318
   gcaacactgt gacgtactgg agggagctta gaggaaaagt attatttcc 49
<210> 319
   <211> 44
   <212> DNA
   <213> synthetic
<400> 319
   gcaacactgt gacgtactgg aggtggtgct gtgccagacg ctgg 44
<210> 320
   <211> 48
   <212> DNA
   <213> synthetic
<400> 320
   gcaacactgt gacgtactgg aggcagatct ttttggctat tgtcttgg 48
<210> 321
   <211> 45
   <212> DNA
   <213> synthetic
<400> 321
   gcaacactgt gacgtactgg agggaaggaa agggcctccc actgc 45
<210> 322
   <211> 46
   <212> DNA
   <213> synthetic
<400> 322
   gcaacactgt gacgtactgg aggcatgaaa aagcatgctg gggagg 46
<210> 323
   <211> 51
   <212> DNA
   <213> synthetic
<400> 323
   gcaacactgt gacgtactgg aggcaaacat aaaaaagctt taatagaagc c 51
<210> 324
   <211> 49
   <212> DNA
   <213> synthetic
<400> 324
   gcaacactgt gacgtactgg aggtcccaac tatgaaaaaa tagaagacg 49
<210> 325
   <211> 46
   <212> DNA
   <213> synthetic
<400> 325
   gcaacactgt gacgtactgg aggcacaaat tagccgggca tggtgg 46
<210> 326
   <211> 49
   <212> DNA
   <213> synthetic
<400> 326
   gcaacactgt gacgtactgg aggcttcctt tactgagtct ttctaaagc 49
<210> 327
   <211> 48
   <212> DNA
   <213> synthetic
<400> 327
   gcaacactgt gacgtactgg aggtgtcctt tgaaatgtag gtatgtgg 48
<210> 328
   <211> 47
   <212> DNA
   <213> synthetic
<400> 328
   gcaacactgt gacgtactgg aggggatctt gcaatactga catctcc 47
<210> 329
   <211> 49
   <212> DNA
   <213> synthetic
<400> 329
   gcaacactgt gacgtactgg aggatttgaa aagaactgaa ggatctacc 49
<210> 330
   <211> 46
   <212> DNA
   <213> synthetic
<400> 330
   gcaacactgt gacgtactgg agggtgagct gagatctcgt ctctgc 46
<210> 331
   <211> 51
   <212> DNA
   <213> synthetic
<400> 331
   gcaacactgt gacgtactgg aggtttgtct gaaacagatt ctaaaagttg 51
<210> 332
   <211> 44
   <212> DNA
   <213> synthetic
<400> 332
   gcaacactgt gacgtactgg agggcaggtg cctgtagtcc cagc 44
<210> 333
   <211> 50
   <212> DNA
   <213> synthetic
<400> 333
   gcaacactgt gacgtactgg agggtttgag cttctaaaat tcatggattc 50
<210> 334
   <211> 46
   <212> DNA
   <213> synthetic
<400> 334
   gcaacactgt gacgtactgg agggtggtag gtcaaaccgc aattcc 46
<210> 335
   <211> 48
   <212> DNA
   <213> synthetic
<400> 335
   gcaacactgt gacgtactgg aggaccaaat cagacatatc agctttgg 48
<210> 336
   <211> 47
   <212> DNA
   <213> synthetic
<400> 336
   gcaacactgt gacgtactgg aggcacagaa cggatcctca ataaagg 47
<210> 337
   <211> 48
   <212> DNA
   <213> synthetic
<400> 337
   gcaacactgt gacgtactgg agggttaact cctcccttct ctttatgg 48
<210> 338
   <211> 41
   <212> DNA
   <213> synthetic
<400> 338
   gtctcaagtg agctacctgg tggcaggagg agccccagag c 41
<210> 339
   <211> 43
   <212> DNA
   <213> synthetic
<400> 339
   gtctcaagtg agctacctgg tggtcctggg gatggttgga tgc 43
<210> 340
   <211> 46
   <212> DNA
   <213> synthetic
<400> 340
   gtctcaagtg agctacctgg tggtgacccc acagagttta cacagc 46
<210> 341
   <211> 42
   <212> DNA
   <213> synthetic
<400> 341
   gtctcaagtg agctacctgg tggagtcagg gcaggctctg cc 42
<210> 342
   <211> 46
   <212> DNA
   <213> synthetic
<400> 342
   tctcaagtg agctacctgg tggtattttg gccccatcca gaaagc 46
<210> 343
   <211> 46
   <212> DNA
   <213> synthetic
<400> 343
   gtctcaagtg agctacctgg tggcacccag agtacagctt tgttcc 46
<210> 344
   <211> 40
   <212> DNA
   <213> synthetic
<400> 344
   gactgcgagg atcaggtctc cgaggagccc cagagcctgc 40
<210> 345
   <211> 43
   <212> DNA
   <213> synthetic
<400> 345
   gactgcgagg atcaggtctc ctggggatgg ttggatgctt acc 43
<210> 346
   <211> 44
   <212> DNA
   <213> synthetic
<400> 346
   gactgcgagg atcaggtctc ccccacagag tttacacagc ttgc 44
<210> 347
   <211> 40
   <212> DNA
   <213> synthetic
<400> 347
   gactgcgagg atcaggtctc ccaggctctg cccactcacc 40
<210> 348
   <211> 42
   <212> DNA
   <213> synthetic
<400> 348
   gactgcgagg atcaggtctc cccatccaga aagcccaaag cc 42
<210> 349
   <211> 47
   <212> DNA
   <213> synthetic
<400> 349
   gactgcgagg atcaggtctc cccagagtac agctttgttc ctcattc 47
<210> 350
   <211> 36
   <212> DNA
   <213> synthetic
<400> 350
   cggacagccc gtcagacgcc agagcctgca agctgc 36
<210> 351
   <211> 41
   <212> DNA
   <213> synthetic
<400> 351
   cggacagccc gtcagacgtg gttggatgct tacccaaaac c 41
<210> 352
   <211> 42
   <212> DNA
   <213> synthetic
<400> 352
   cggacagccc gtcagacgca gagtttacac agcttgcctt gg 42
<210> 353
   <211> 38
   <212> DNA
   <213> synthetic
<400> 353
   cggacagccc gtcagacgct ctgcccactc acccctgc 38
<210> 354
   <211> 40
   <212> DNA
   <213> synthetic
<400> 354
   cggacagccc gtcagacgca gaaagcccaa agccaacagg 40
<210> 355
   <211> 44
   <212> DNA
   <213> synthetic
<400> 355
   cggacagccc gtcagacgag tacagctttg ttcctcattc tgac 44
<210> 356
   <211> 39
   <212> DNA
   <213> synthetic
<400> 356
   ggaataggga agaaacgcag gccctgcaag ctgccgtgc 39
<210> 357
   <211> 45
   <212> DNA
   <213> synthetic
<400> 357 agaaacgcag gcgcttaccc aaaaccatac agtgg 45
<210> 358
   <211> 46
   <212> DNA
   <213> synthetic
<400> 358
   ggaataggga agaaacgcag gcacagcttg ccttgggtgt tataac 46
<210> 359
   <211> 41
   <212> DNA
   <213> synthetic
<400> 359
   ggaataggga agaaacgcag gcactcaccc ctgcaggcac c 41
<210> 360
   <211> 44
   <212> DNA
   <213> synthetic
<400> 360
   ggaataggga agaaacgcag gcagcccaaa gccaacagga gtcc 44
<210> 361
   <211> 46
   <212> DNA
   <213> synthetic
<400> 361
   ggaataggga agaaacgcag gcctttgttc ctcattctga ctgagc 46
<210> 362
   <211> 21
   <212> DNA
   <213> synthetic
<400> 362
   gactgcgagg atcaggtctc c 21
<210> 363
   <211> 18
   <212> DNA
   <213> synthetic
<400> 363
   cggacagccc gtcagacg 18
<210> 364
   <211> 22
   <212> DNA
   <213> synthetic
<400> 364
   ggaataggga agaaacgcag gc 22
<210> 365
   <211> 42
   <212> DNA
   <213> synthetic
<400> 365
   gcagtacaaa caacgcacag cgctgccacc ctccacagtc cc 42
<210> 366
   <211> 40
   <212> DNA
   <213> synthetic
<400> 366
   gcagtacaaa caacgcacag cggccaagac catgcatgcg 40
<210> 367
   <211> 43
   <212> DNA
   <213> synthetic
<400> 367
   gcagtacaaa caacgcacag cgcccaggga caaagagact ccc 43
<210> 368
   <211> 48
   <212> DNA
   <213> synthetic
<400> 368
   gcagtacaaa caacgcacag cgcaggaagc agacagtctt ctagttcc 48
<210> 369
   <211> 45
   <212> DNA
   <213> synthetic
<400> 369
   gcagtacaaa caacgcacag cgtgcctgta atcccaacac tttgg 45
<210> 370
   <211> 41
   <212> DNA
   <213> synthetic
<400> 370
   gcagtacaaa caacgcacag cgtccctctg gccaggatgg 41
<210> 371
   <211> 44
   <212> DNA
   <213> synthetic
<400> 371
   gcagtacaaa caacgcacag cgatggggaa tgggagtagg aagc 44
<210> 372
   <211> 44
   <212> DNA
   <213> synthetic
<400> 372
   gcagtacaaa caacgcacag cgcagatcag ttctcccctc cagc 44
<210> 373
   <211> 48
   <212> DNA
   <213> synthetic
<400> 373
   gcagtacaaa caacgcacag cgacaaaaaa gaaacatgct cagagagg 48
<210> 374
   <211> 44
   <212> DNA
   <213> synthetic
<400> 374
   gcagtacaaa caacgcacag cgtggtggca tgcatctgta gtcc 44
<210> 375
   <211> 48
   <212> DNA
   <213> synthetic
<400> 375
   gcagtacaaa caacgcacag cgaggtgctc tatagatgtt agcatccc 48
<210> 376
   <211> 44
   <212> DNA
   <213> synthetic
<400> 376
   gcagtacaaa caacgcacag cgccaggaca ggatggagat ctgg 44
<210> 377
   <211> 46
   <212> DNA
   <213> synthetic
<400> 377
   gcagtacaaa caacgcacag cgagggaacc tgtgcattat ccttgc 46
<210> 378
   <211> 47
   <212> DNA
   <213> synthetic
<400> 378
   gcagtacaaa caacgcacag cgcagaagtc ttgctttaag gaggagg 47
<210> 379
   <211> 44
   <212> DNA
   <213> synthetic
<400> 379
   gcagtacaaa caacgcacag cggggtacgt gaaactcacc aagg 44
<210> 380
   <211> 42
   <212> DNA
   <213> synthetic
<400> 380
   gcagtacaaa caacgcacag cgcagagtgt ggcaagcaag gg 42
<210> 381
   <211> 50
   <212> DNA
   <213> synthetic
<400> 381
   gcagtacaaa caacgcacag cgaacatttt aaaggtacaa ataacgtggg 50
<210> 382
   <211> 44
   <212> DNA
   <213> synthetic
<400> 382
   gcagtacaaa caacgcacag cgtagggagc aacagccatt aagc 44
<210> 383
   <211> 42
   <212> DNA
   <213> synthetic
<400> 383
   gcagtacaaa caacgcacag cgggtgcact gtccagctct gg 42
<210> 384
   <211> 43
   <212> DNA
   <213> synthetic
<400> 384
   gcagtacaaa caacgcacag cgactctcgc tgaactcgcc tgg 43
<210> 385
   <211> 42
   <212> DNA
   <213> synthetic
<400> 385
   gcagtacaaa caacgcacag cgctcggtct ctggtggtac gc 42
<210> 386
   <211> 41
   <212> DNA
   <213> synthetic
<400> 386
   gcagtacaaa caacgcacag cggcaagagg tccgagctgg g 41
<210> 387
   <211> 48
   <212> DNA
   <213> synthetic
<400> 387
   gcagtacaaa caacgcacag cgggaagaag tgaaacaaga gatgaagg 48
<210> 388
   <211> 45
   <212> DNA
   <213> synthetic
<400> 388
   gcagtacaaa caacgcacag cgcccagaga acaaaccgga ttagg 45
<210> 389
   <211> 45
   <212> DNA
   <213> synthetic
<400> 389
   gcagtacaaa caacgcacag cgcccttcaa ccttctccaa tctgc 45
<210> 390
   <211> 43
   <212> DNA
   <213> synthetic
<400> 390
   gcagtacaaa caacgcacag cgcccatgtc cagtggttta ggg 43
<210> 391
   <211> 44
   <212> DNA
   <213> synthetic
<400> 391
   gcagtacaaa caacgcacag cggagattgg tgggagacag atgg 44
<210> 392
   <211> 45
   <212> DNA
   <213> synthetic
<400> 392
   gcagtacaaa caacgcacag cgcttctcag ctcaaagttc cagcg 45
<210> 393
   <211> 44
   <212> DNA
   <213> synthetic
<400> 393
   gcagtacaaa caacgcacag cggaatggga gagatgacca gagg 44
<210> 394
   <211> 42
   <212> DNA
   <213> synthetic
<400> 394
   gcagtacaaa caacgcacag cgaagggcaa gggggtatgt gg 42
<210> 395
   <211> 43
   <212> DNA
   <213> synthetic
<400> 395
   gcagtacaaa caacgcacag cgggaaggaa gcatgggaac acc 43
<210> 396
   <211> 45
   <212> DNA
   <213> synthetic
<400> 396
   gcagtacaaa caacgcacag cgccatcaat gctctgtctg tctgg 45
<210> 397
   <211> 41
   <212> DNA
   <213> synthetic
<400> 397
   gcagtacaaa caacgcacag cggtgccgtg actgtgcttg g 41
<210> 398
   <211> 45
   <212> DNA
   <213> synthetic
<400> 398
   gcagtacaaa caacgcacag cgacatccca ttgacctcat caagc 45

## Description

### FIELD OF THE INVENTION

The present invention relates generally to a method of amplifying a nucleic acid region of interest and, more particularly, to a method of amplifying a nucleic acid region of interest using a PCR method designed to minimise the generation of amplicons from primers which have bound to nucleic acid regions other than the specific region of interest. The method of the present invention is based on the determination that by rendering inefficient the functionality of either the forward primer or the reverse primer, the rate of amplification of irrelevant nucleic acid regions can be reduced relative to amplification of the region of interest. The provision of a selective means of amplifying a nucleic acid region of interest is useful in a range of applications including, but not limited to, the diagnosis and/or monitoring of disease conditions which are characterised by specific gene sequences, the characterisation or analysis of gene regions of interest, the identification or characterisation of DNA breakpoint regions and the isolation of gene sequences of interest where only the nucleotide sequence at one end of the gene sequence of interest is known.

### BACKGROUND OF THE INVENTION

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Bibliographic details of the publications referred to by author in this specification are collected alphabetically at the end of the description.

The polymerase chain reaction (PCR) is a technique which is utilised to amplify specific regions of a DNA strand. This may be a single gene, just a part of a gene or a non-coding sequence. Most PCR methods typically amplify DNA fragments of up to 10 kilo base pairs (kb), although some techniques allow for amplification of fragments up to 40 kb in size (Cheng et al., 1994, Proc Natl Acad Sci. 91:5695-5699).

PCR, as currently practiced, requires several basic components (Sambrook and Russel, 2001, Molecular Cloning: A Laboratory Manual, 3rd Ed.). These components are:
- a DNA template which contains the region of the DNA fragment to be amplified;
- primers, which are complementary to the DNA regions at the 5' and 3' ends of the DNA region that is to be amplified;
- a DNA polymerase (e.g. *Taq polymerase* or another thermostable DNA polymerase with a temperature optimum at around 70°C), used to synthesize a DNA copy of the region to be amplified; and
- Deoxynucleotide triphosphates (dNTPs) from which the DNA polymerase builds the new DNA.

PCR is carried out in small reaction tubes (0.2-0.5 ml volumes), containing a reaction volume typically of 15-100µl, which are inserted into a thermal cycler. This machine heats and cools the reaction tubes within it to the precise temperature required for each step of the reaction. Most thermal cyclers comprise heated lids to prevent condensation on the inside of the reaction tube caps. Alternatively, a layer of oil may be placed on the reaction mixture to prevent evaporation.

Accordingly, PCR is a method that allows exponential amplification of DNA sequences within a longer DNA molecule. The reaction involves a number of cycles of amplification, and in each cycle the template for each molecular reaction is either a strand of genomic DNA or a strand of DNA synthesised in a preceding cycle. Each PCR cycle involves the following steps
- denaturation by heat to separate the 2 strands of double-stranded DNA molecules
- hybridisation of the upstream and downstream primers to their complementary sequences
- extension of the primers by the DNA polymerase to produce a complementary copy of the template sequence

Typically the PCR reagents and conditions are chosen so that denaturation, hybridisation and extension occur at close to maximum efficiency and as a result the amount of the desired sequence increases with each cycle by a factor of close to 2. Substantial amplification occurs by the end of the PCR eg a 30 cycle PCR will result in amplification of the original template by a factor of almost 2³⁰ (1,000,000,000). This degree of amplification facilitates detection and analysis of the amplified product.

Various PCR based assays have been disclosed for the amplification of nucleic acids. For instance Zhu et al. (Multiplex Asymmetric PCR-Based Oligonucleotide Microarray for Detection of Drug Resistance Genes Containing Single Mutations in Enterobacteriaceae, Antimicrobial Agents and Chemotherapy, 2007, pp. 3707-3713) discloses a modified PCR for generating large amounts of single stranded DNA. WO 2006/094360 discloses a modified PCR method for amplifying multiple nucleic acids in a single closed-tube, comprising two rounds of amplification, wherein the first round uses primers suitable for performing exhaustive PCR.

Other nucleic acid amplification techniques, such as the Ligase Chain Reaction (LCR) or the Nucleic Acid Sequence Based Reaction (NASBA), are also used to amplify a desired sequence in DNA. The reaction strategies differ from that of the PCR but they also use primers that hybridise to the 2 ends of the target sequence and again, the reaction is typically performed to ensure that each step, including hybridisation, occurs at or close to maximum efficiency. Although the ensuing discussion is largely directed towards PCR, the concepts equally apply to other amplification techniques.

After a number of cycles of amplification, the PCR product can be analyzed in various ways, most commonly by gel electrophoresis. In its simplest form this method of analysis is semi-quantitative. The amount of product is not closely related to the amount of input DNA, thereby making this type of PCR a qualitative tool for detecting the presence or absence of a particular DNA.

In order to measure messenger RNA (mRNA), the method uses reverse transcriptase to initially convert mRNA into complementary DNA (cDNA) which is then amplified by PCR and analyzed by agarose gel electrophoresis. In many cases this method has been used to measure the levels of a particular mRNA under different conditions. However, this method is actually even less quantitative than the PCR of DNA because of the extra reverse transcriptase step.

In order to provide quantitation capabilities, real-time PCR was developed. This procedure follows the general pattern of PCR, but the amplified DNA is quantified during each cycle. Two common methods of quantification are the use of fluorescent dyes that intercalate with double-stranded DNA and modified DNA oligonucleotide primers or probes the fluorescence of which changes during one of the steps of the PCR. Frequently, real-time polymerase chain reaction is combined with reverse transcriptase polymerase chain reaction to quantify low abundance messenger RNA (mRNA), enabling a researcher to quantify relative gene expression at a particular time or in a particular cell or tissue type.

### (i) Real-time PCR using dyes binding to double-stranded DNA

A DNA-binding dye binds to all double-stranded (ds)DNA in a PCR reaction, causing increased fluorescence of the dye. An increase in DNA product during PCR therefore leads to an increase in fluorescence intensity which is measured at each cycle, thus allowing DNA concentrations to be quantified. Like other real-time PCR methods, the values obtained do not have absolute units associated with them (i.e. mRNA copies/cell). Accordingly, a comparison of a measured DNA/RNA sample to a standard dilution will only give a fraction or ratio of the sample relative to the standard, allowing only relative comparisons between different tissues or experimental conditions. To ensure accuracy in the quantification, it is usually necessary to normalize expression of a target gene to a stably expressed gene. This can correct for possible differences in RNA quantity or quality across experimental samples.

### (ii) Fluorescent reported sequence methods

A number of different methods using fluorescent reporter primers or probes have been developed and they tend to be more accurate and reliable than use of DNA binding dyes. They use one or more DNA primers or probes to quantify only the DNA to which the primer or probe hybridises. Use of a reporter probe significantly increases specificity and may allow quantification even in the presence of some non-specific DNA amplification. Use of sequence-specific primers or probes allows for multiplexing - assaying for several different amplified products in the same reaction by using specific sequences or probes with different-coloured labels, provided that all targets are amplified with similar efficiency.

In terms of quantitation, relative concentrations of DNA present during the exponential phase of the reaction are determined by plotting fluorescence against cycle number on a logarithmic scale. A threshold for increase of fluorescence above background or decrease below background (depending on the precise method) is determined. The cycle at which the fluorescence from a sample crosses the threshold is called the cycle threshold, Cₜ. Since the quantity of DNA doubles every cycle during the exponential phase, relative amounts of DNA can be calculated, e.g. a sample whose Cₜ is 3 cycles earlier than another's has 2³ = 8 times more template (assuming that the amount of amplified DNA doubles with each cycle).

Amounts of DNA are then determined by comparing the results to a standard curve produced by serial dilutions (e.g. undiluted, 1:4, 1:16, 1:64) of a known amount of DNA.

However, one of the limitations of PCR relates to the fact that primers can, in some situations, bind to more than one region of a DNA sample, thereby potentially leading to the generation of amplified sequences which are unrelated to the DNA sequence of interest. Binding to multiple regions may occur in a number of situations which include but are not limited to
1. Non-specific binding of the primer owing to, for example, the primer being very short or degenerate or of a design which favours non-specific binding or subject to PCR conditions which favour non-specific binding.
2. The sequence to which a primer binds naturally occurs in many regions of the genome. Sequences belonging to families such as *alu* or *line* are widely dispersed and, although individual sequences may vary slightly, their homology is such that a primer binding to one member of a family is likely to bind to numerous other members.
3. The sequence to which a primer binds has been introduced into many regions of the genome. This may occur, for example, if the DNA has been digested by a restriction enzyme and a common sequence has been ligated at the sites of digestion.
4. The presence of multiple primers in the reaction. By chance, one primer may bind so as to act as a forward primer and another may bind so as to act as a reverse primer. The probability of occurrence of this phenomenon will increase as the number of primers increases.

As a consequence of one of these situations, it may be the case that one primer binds so as to act as a forward primer and the same or another primer binds so as to act as a reverse primer. If the binding sites are sufficiently close, non-specific amplification may occur. If one considers that the capacity of PCR to amplify over one billion fold also increases the possibility of amplifying the wrong DNA sequence over one billion times, the importance of minimising this possibility becomes clear.

Accordingly, there is an ongoing interest and need to develop means of overcoming this problem. The usual approach is nested PCR. In this method two pairs of PCR primers are used for a single locus. The first pair amplifies the locus as seen in any PCR experiment. The second pair of primers (nested primers) bind within the first PCR product and produce a second PCR product that will be shorter than the first one. The logic behind this strategy is that if an unwanted locus was also amplified, the probability is very low that it would also be amplified a second time by a second pair of primers. However nested PCR is only of value if some or all of the DNA sequence internal to the first pair of primers is known so that 1 or more internal primers which provide additional specificity can be synthesised.

In work leading up to the present invention, another method of minimising unwanted amplification events has been developed. Specifically, it has been determined that if one of the two PCR primers (e.g. primer 2) is designed or used such that it hybridises inefficiently and comprises a nucleic acid tag which can itself lead to a binding site for a third (tag) primer, a "bottleneck" in amplification will occur. The bottleneck occurs because efficient amplification of the desired product will only commence after primer 2 has hybridised and extended, and this process is inefficient. In subsequent cycles, exponential amplification initiated from the templates so produced is efficient, being mediated by efficient hybridisation and extension of primer 1 in one direction and efficient hybridisation and extension of the tag primer in the other direction. However, for undesired amplicons generated from sequences for which primer 2 can act as both a forward and a reverse primer, the bottleneck during the PCR will be much more severe. For such sequences, efficient amplification by the tag primer would only occur after 2 sequential and inefficient hybridisations of primer 2 have occurred, one in the forward and one in the reverse direction. Thus, by deliberately ensuring that hybridisation of one primer in the PCR is rendered inefficient, one can select against amplification of sequences for which that primer acts as both a forward and reverse primer, and favour amplification of sequences which are amplified from one end by that primer and from the other end by an efficiently hybridising primer.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

One aspect of the present invention provides a method of amplifying a nucleic acid region of interest, said method comprising:
(i) contacting a nucleic acid sample with:
   (a) one or more forward primers directed to said region of interest; and
   (b) one or more reverse primers directed to said region of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the nucleic acid sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified nucleic acid of step (ii) with a third primer which is directed to part or all of the sequence which is complementary to that of the oligonucleotide tag of step (i), wherein said third primer is efficient in the amplification of step (iv); and
(iv) amplifying the nucleic acid sample of step (iii) using the third primer of step (iii) and the efficient primers of group (a) or group (b) of step (i) and (ii), wherein said third primer and efficient primers of group (a) or group (b) hybridise and extend efficiently.

Another aspect of the present invention provides a method of amplifying a DNA region of interest, said method comprising:
(i) contacting a DNA sample with:
   (a) one or more forward primers directed to said region of interest; and
   (b) one or more reverse primers directed to said region of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

In yet another aspect the present invention provides a method of amplifying a gene or gene fragment of interest, said method comprising:
(i) contacting a DNA sample with:
   (a) one or more forward primers directed to said gene or gene fragment of interest; and
   (b) one or more reverse primers directed to said gene or gene fragment of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

In still another aspect, there is provided a method of amplifying a chromosomal gene translocation breakpoint region of interest, said method comprising:
(i) contacting a DNA sample with:
   (a) one or more forward primers directed to said chromosomal gene translocation breakpoint region of interest; and
   (b) one or more reverse primers directed to said chromosomal gene translocation breakpoint region of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

In yet still another aspect, there is provided a method of amplifying a DNA region of interest, said method comprising:
(i) contacting a DNA sample with:
   (a) one or more forward primers directed to said region of interest; and
   (b) one or more reverse primers directed to said region of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii) and wherein said functionally inefficient primer group is the primer group which has the potential to hybridise promiscuously to non-target DNA regions;
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

In still yet another aspect, there is provided a method of amplifying a DNA region of interest, said method comprising:
(i) contacting a DNA sample with:
   (a) one or more forward primers directed to said region of interest; and
   (b) one or more reverse primers directed to said region of interest
   wherein the primers of either group (a) or group (b) hybridise inefficiently in the amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

A further aspect of the present invention provides a method of amplifying a DNA region of interest, said method comprising:
(i) contacting a DNA sample with:
   (a) one or more forward primers directed to said region of interest; and
   (b) one or more reverse primers directed to said region of interest
   wherein the primers of one of group (a) or group (b) hybridise inefficiently in the PCR amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of PCR using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) by PCR using primers (a) and (b) wherein the tagged primers of (a) or (b) hybridise and extend inefficiently and the other primers of (a) or (b) hybridise and extend efficiently.

A further method of amplifying a DNA region of interest is disclosed herein, said method comprising:
(i) contacting a DNA sample with:
   (a) one or more forward primers directed to said region of interest; and
   (b) one or more reverse primers directed to said region of interest
   wherein the primers of either group (a) or group (b) hybridise inefficiently and are both functionally inefficient and are operably linked at their 5' end to an oligonucleotide tag;
(ii) amplifying the DNA sample of step (i) through at least two cycles of amplification;
(iii) contacting the amplified DNA of step (ii) with a primer which is directed to part or all of the sequence which is complementary to that of the oligonucleotide tag of step (i);
(iv) amplifying the DNA sample of step (iii); and
(v) isolating and/or analysing said amplified DNA.

### BRIEF DESCRIPTION OF THE DRAWINGS

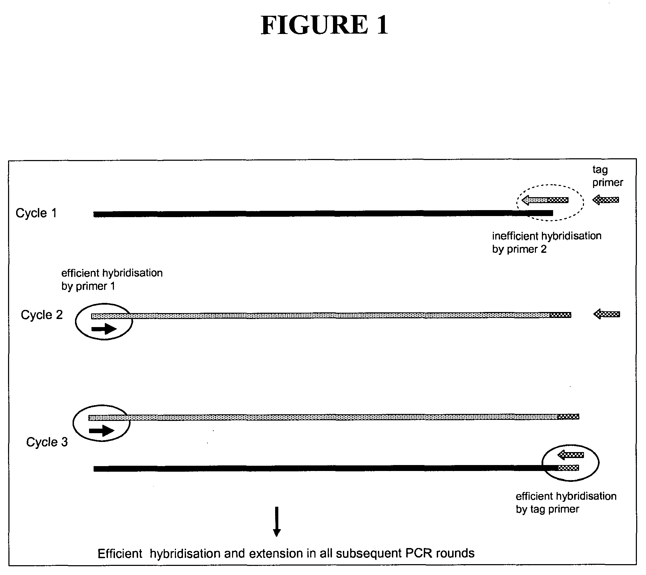

**Figure 1** is a schematic representation depicting, for amplification of the sequence of interest, the 2 events in 2 (not necessarily successive) PCR cycles which must occur before efficient amplification occurs. The first is when the inefficient primer 2 hybridises and extends to form an antisense strand, the second is when the efficient primer 1 hybridises to the resultant antisense strand and extends to form a sense strand containing the binding sequence for the tag primer. Efficient amplification in subsequent cycles then occurs primed by primer 1 and the tag primer.

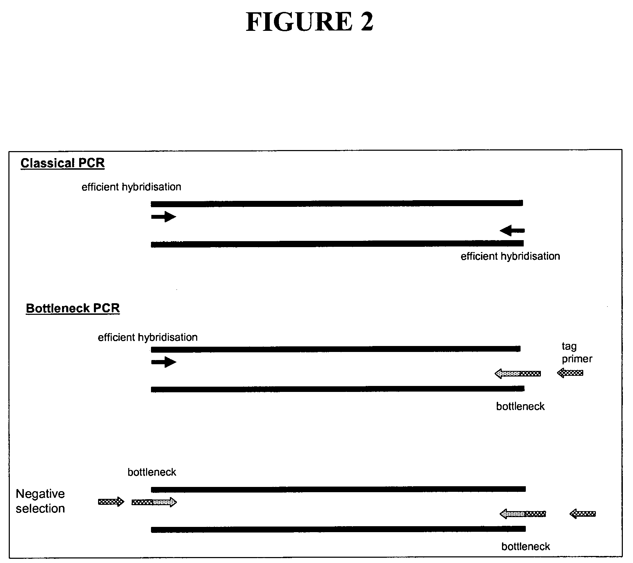

**Figure 2** is a schematic representation of negative selection during bottleneck PCR. In classical PCR there is efficient hybridisation and extension at both upstream and downstream primer sites. In Bottleneck PCR there is a bottleneck owing to inefficient hybridisation at either one or both of the primer-binding sites. This results in negative selection for targets for which there is a bottleneck at each end as compared to those for which there is a bottleneck at only one end.

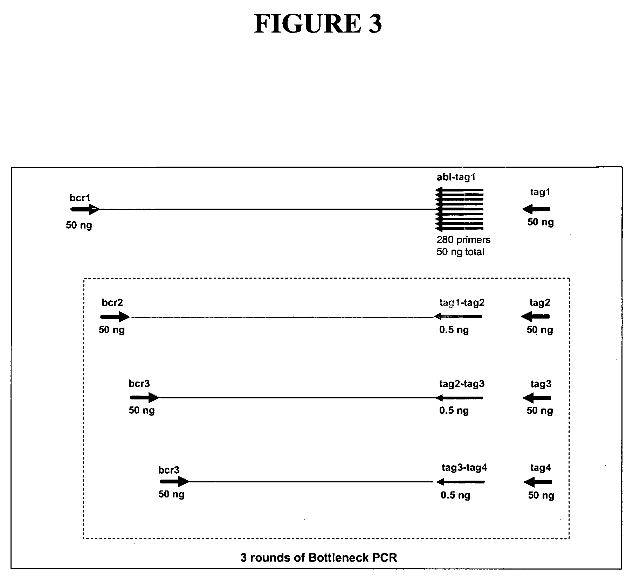

**Figure 3** is a schematic representation of the experimental design for enrichment of *BCR-ABL* breakpoint sequence using Bottleneck PCR. The second, third and fourth PCR rounds were bottleneck PCRs, as the hybrid-tag downstream primers were present at a low concentration, resulting in inefficient hybridisation and extension. There was also some incidental bottleneck effect in the first round owing to the low concentration of each downstream *ABL* primer. In a typical experiment, the first 3 rounds would each involve 20 PCR cycles and the fourth final round would involve 20-35 cycles.

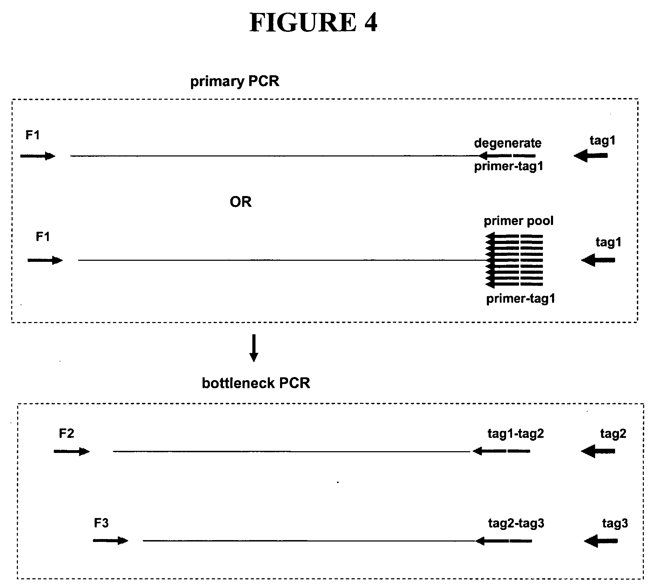

**Figure 4** is a schematic representation of the experimental design for enrichment of a desired product using bottleneck PCR. The primary PCR involves a forward gene-specific primer (F1) and either a degenerate (universal) reverse primer, if gene walking is being performed, or a pool of reverse primers, if amplification across a translocation breakpoint is being performed. The primary PCR can be designed as a bottleneck PCR by rendering the reverse primer(s) inefficient and, when a pool of reverse primers is used, actually performs as a bottleneck PCR owing to the low concentration of each reverse primer. The second, and third PCR rounds are definitive bottleneck PCRs. They involve the same or a nested forward primer and a low concentration of the hybrid-tag reverse primer, which results in inefficient hybridisation and extension of this primer. In a typical experiment, the first 2 or 3 rounds would each involve 20 PCR cycles and the final round would involve 20-35 cycles.

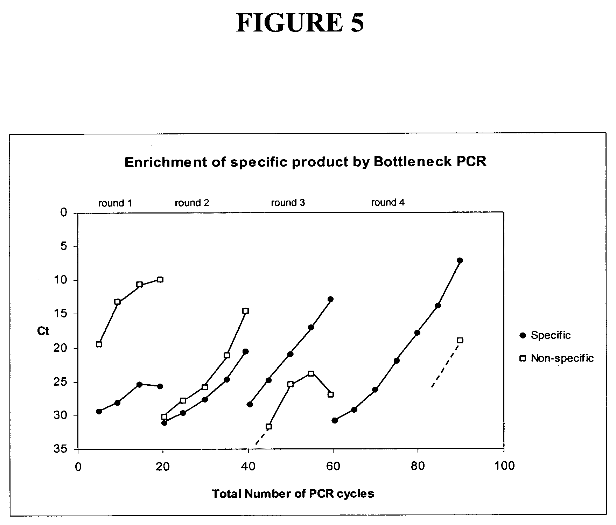

**Figure 5** is a graphical representation of the enrichment of specific product by selection against non-specific products. The specific product was the *BCR-ABL* translocation sequence and the non-specific products were anonymous genomic sequences to which one or more of the *ABL* primers hybridised at each end resulting in amplification. DNA from a patient with the *BCR-ABL* translocation was amplified in round 1 with an upstream *BCR* primer and 282 downstream tagged *ABL* primers. Three successive rounds of bottleneck PCR were then performed, each using an upstream *BCR* primer and a downstream linking hybrid tag*ᵢ* - tag_{*i*+1} primer and a tag_{*i*+1} primer. Amplification during each round was quantified, using sequence-specific primers for the *BCR-ABL* translocation and the tag primer for the non-specific products. As shown by the Ct values for the 2 products, non-specific product greatly predominates in the first round but its relative amount decreases with each round so that specific product predominates in rounds 3 and 4. The degree of enrichment observed between rounds 2 and 1 and between rounds 3 and 2 suggests that the tag-tag primers were acting at approximately 1% of maximum efficiency.

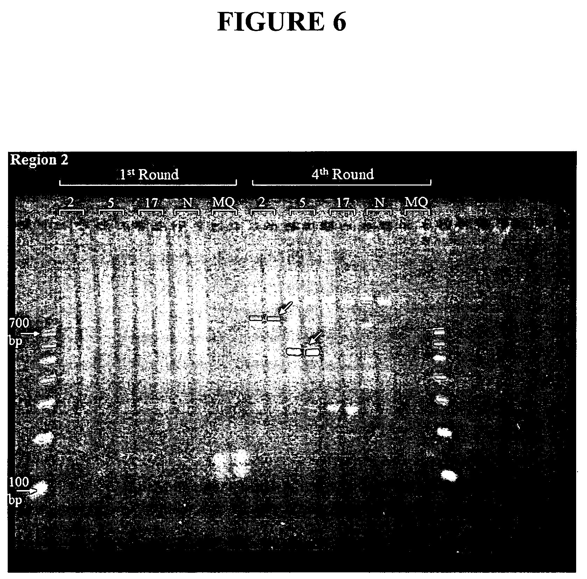

**Figure 6** is an image of the selective effect of bottleneck PCR in facilitating isolation of translocation breakpoint regions. Lanes 2, 5 and 17 show results of amplification with DNA from 3 patients with chronic myeloid leukaemia, lane N shows results with DNA from a normal control and lane MQ shows results from a water control. In the first cycle of PCR amplification, all samples were amplified with 1 upstream primer directed against *BCR* and 282 primers directed against the *ABL* gene. For one set of amplifications, the first round was continued for 45 cycles (1^{st} round), whereas for the other set of amplifications the PCR was stopped after 20 cycles and a 1/100 aliquot was used to perform 3 further rounds of Bottleneck PCR (4^{th} cycle). The 1^{st} round results show a broad smear of heterogeneous products which have been amplified owing to priming at both ends by the *ABL* primers. The 4^{th} round products are much less complex and homogeneous bands can be seen. There is one non-specific band seen in all samples, and in patients 2 and 5 the band due to amplification of the *BCR-ABL* translocation can be seen (arrows).

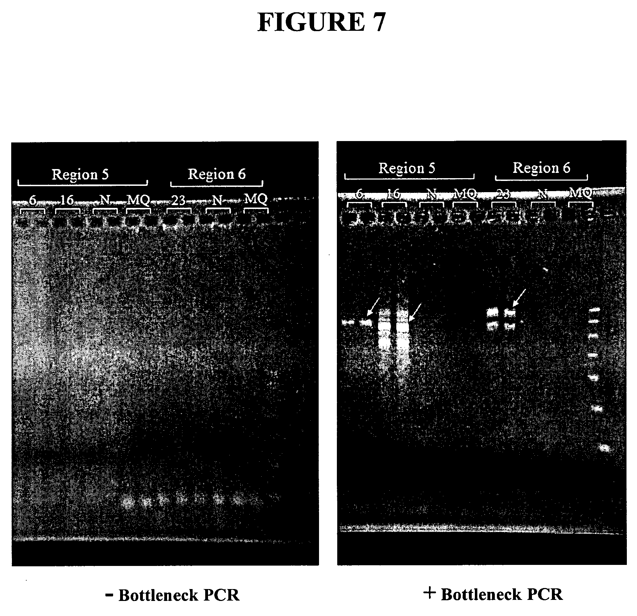

**Figure 7** is another image of the selective effect of bottleneck PCR. Lanes 6, 16 and 23 show results of amplification with DNA from 3 patients with chronic myeloid leukaemia, lane N shows results with DNA from a normal control and lane MQ shows results from a water control. In the first round of PCR amplification, all samples were amplified with 1 upstream primer directed against *BCR* and 282 primers directed against the *ABL* gene; in the second round, all samples were amplified with 1 nested upstream primer directed against *BCR* and 282 nested primers directed against the *ABL* gene. When Bottleneck PCR was not performed, the second round was continued for 45 cycles, whereas, when it was performed, the second round PCR was stopped after 20 cycles and a 1/100 aliquot was used to perform 2 further rounds of bottleneck PCR. This resulted in preferential amplification of the *BCR-ABL* sequence in the 3 patients (indicated by arrows).

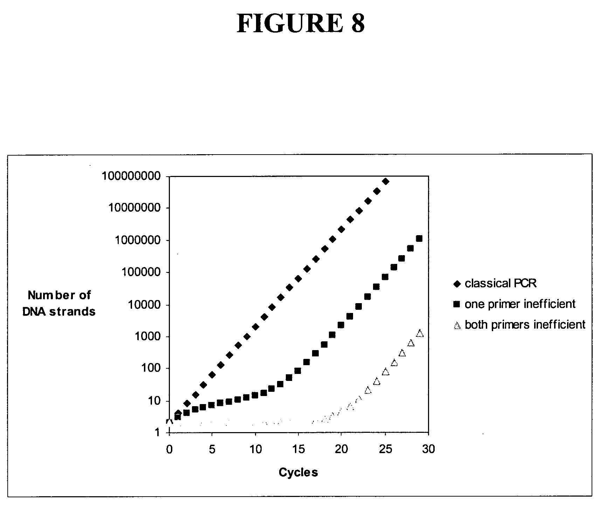

**Figure 8** is a graphical representation depicting a model of amplification produced in a classical PCR or in a single-round bottleneck PCR in which either one primer or both primers are inefficient. The model was produced using the Excel spreadsheet. In the example shown, the starting target for each PCR is one DNA duplex, comprising a sense and an antisense strand, and the probability of hybridising and extending during each cycle is 1 for the efficient primer(s) and 0.001 for the inefficient primer(s). The bottleneck before exponential amplification predominates is evident. After any given number of PCR cycles, the target amplified by one efficient and one inefficient primer has increased approximately 1000-fold compared to the target amplified by the two inefficient primers.

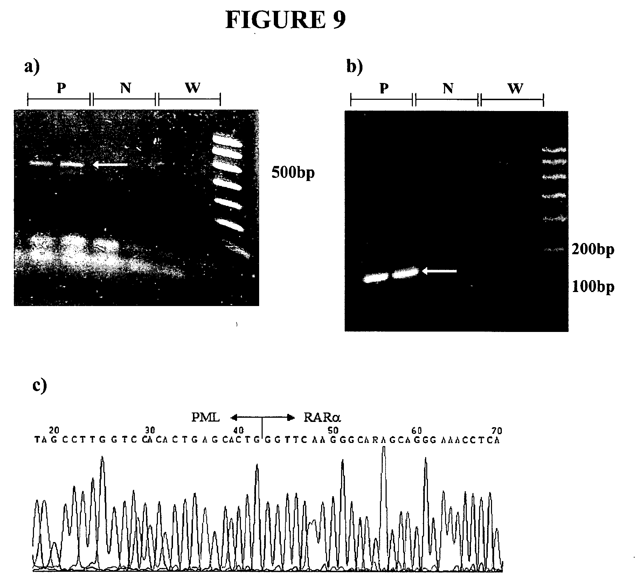

**Figure 9** shows images from an experiment using bottleneck PCR to isolate the *PML-RARα* translocation breakpoint from a patient with acute promyelocytic leukemia.
   a) The patient DNA was amplified using multiple RARα primers and a single PML primer and then 2 rounds of bottleneck PCR were performed. The Figure shows the amplified DNA electrophoresed on a 2% agarose gel. P is patient DNA, N is the normal DNA and W is the water control. The DNA ladder shows bands 700bp to 100bp descending from top to bottom. The patient band can be seen at approximately 500bp.
   b) To confirm that the breakpoint had been isolated, the breakpoint sequence was used to design RARα and PML primers spanning the breakpoint, and the patient DNA was amplified for one round using these primers. The Figure shows the amplified DNA electrophoresed on a 2% agarose gel. P is the patient DNA, N is the normal DNA and W is the water control. The DNA ladder shows bands 700bp to 100bp descending from top to bottom. The confirmatory patient band can be seen at approximately 150bp.
   c) The sequence chromatogram obtained from the amplified band shown on the gel in section a of the figure. The breakpoint between the PML and RARα is shown.

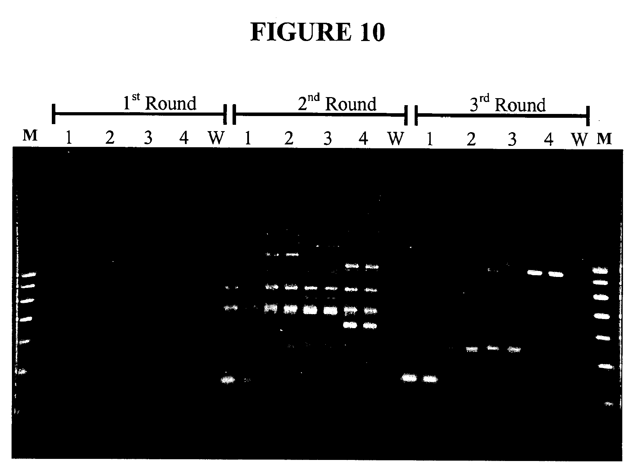

**Figure 10** shows images illustrating the use of bottleneck PCR for gene walking, using a "universal" degenerate primer, along the Myocillin gene using 4 different degenerate primers (lanes 1-4), which were identical except that their fifth most 3' base was either A, G, C or T. The first round is the primary PCR and the second and third rounds are bottleneck PCRs. The decrease in complexity with serial bottleneck PCRs is apparent. Sequencing of the predominant band in each of the four amplifications showed the expected Myocillin gene sequence Lane M is a DNA ladder with products from 700-100 bp at 100bp intervals, W is the water control with no DNA.

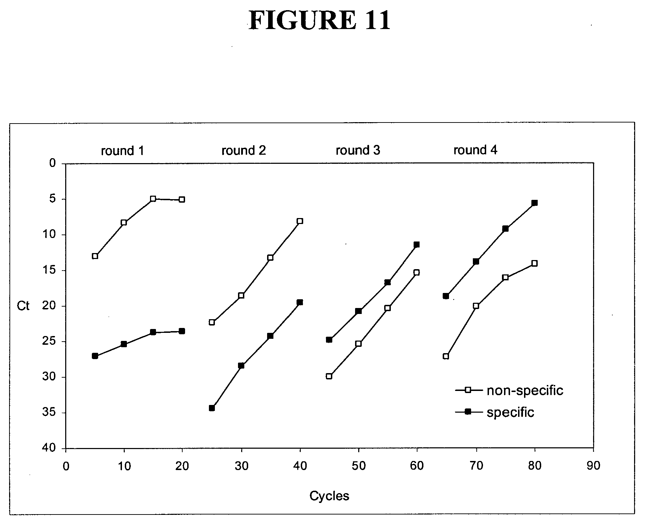

**Figure 11** is a graphical illustration of progressive selection against non-specific amplification products by bottleneck PCR. The experiment studied walking along the APC gene. The primary PCR used 20 cycles with one gene-specific and one degenerate primer and was followed by 3 rounds of bottleneck PCR each of 20 cycles. Amplification products were sampled every 5 cycles and specific and non-specific products were assayed in a "read-out" PCR. A decreasing Ct indicates increasing product. Specific product starts to predominate after 1 bottleneck PCR.

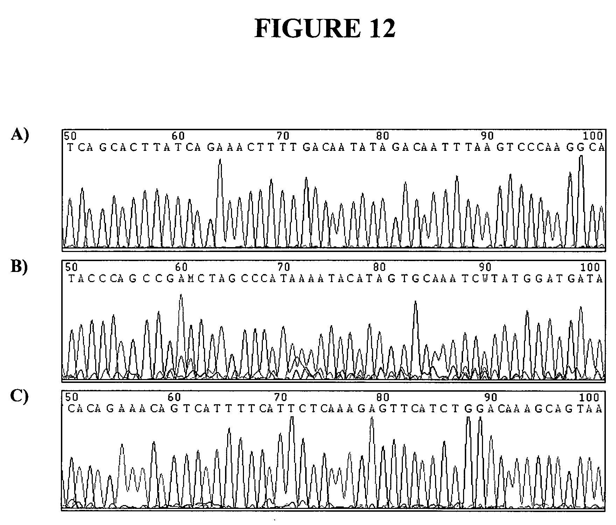

**Figure 12** shows images of *APC* gene sequences from a three round genewalking experiment. A primary PCR using an APC gene-specific primer and a degenerate reverse primer was followed by 2 rounds of bottleneck PCR and the amplification product was sequenced directly, without electrophoresis. Three APC sequences in the forward direction, using different sequencing primers, are shown. The sequencing primer used to obtain **A** was the same as that used in the third PCR round. The sequencing primers used to obtain **B** and **C** were 732 and 1302 bps respectively from the most 5' prime primer. The total length of readable sequence was approximately 1.5 kb.

### DETAILED DESCRIPTION OF THE INVENTION

The present invention is predicated, in part, on the determination that the incidence of unwanted amplicon generation, deriving from the binding of primers to DNA regions other than the region of interest, can be minimised by tagging and rendering functionally inefficient the primer which is likely to result in primer hybridisation and extension at a site which is not the site of interest. The method of the present invention therefore provides a simple and efficient means of amplifying a nucleic acid region of interest.

Accordingly, one aspect of the present invention provides a method of amplifying a nucleic acid region of interest, said method comprising:
(i) contacting a nucleic acid sample with:
   (a) one or more forward primers directed to said region of interest; and
   (b) one or more reverse primers directed to said region of interest
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the nucleic acid sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified nucleic acid of step (ii) with a third primer which is directed to part or all of the sequence which is complementary to that of the oligonucleotide tag of step (i), wherein said third primer is efficient in the amplification of step (iv); and
(iv) amplifying the nucleic acid sample of step (iii) using the third primer of step (iii) and the efficient primers of group (a) or group (b) of step (i) and (ii), wherein said third primer and efficient primers of group (a) or group (b) hybridise and extend efficiently.

Reference to a nucleic acid "region of interest" should be understood as a reference to any region of DNA or RNA which is sought to be amplified. This may be a gene or part of a gene. To this end, reference to "gene" should be understood as a reference to a DNA molecule which codes for a protein product, whether that be a full length protein or a protein fragment. In terms of chromosomal DNA, the gene will include both intron and exon regions. However, to the extent that the DNA of interest is cDNA, such as might occur if the DNA of interest is vector DNA or reverse transcribed mRNA, there may not exist intron regions. Such DNA may nevertheless include 5' or 3' untranslated regions. Accordingly, reference to "gene" herein should be understood to encompass any form of DNA which codes for a protein or protein fragment including, for example, genomic DNA and cDNA. The subject nucleic acid region of interest may also be a non-coding portion of genomic DNA which is not known to be associated with any specific gene (such as the commonly termed "junk" DNA regions). It may be any region of genomic DNA produced by recombination, either between 2 regions of genomic DNA or 1 region of genomic DNA and a region of foreign DNA such as a virus or an introduced sequence. It may be a region of a partly or wholly synthetically or recombinantly generated nucleic acid molecule. The subject nucleic acid sequence of interest may also be a region of DNA which has been previously amplified by any nucleic acid amplification method, including PCR (i.e. it has been generated by an amplification method).

The subject "nucleic acid" region may be DNA or RNA or derivative or analogue thereof. Where the region of interest is a DNA sequence which encodes a proteinaceous molecule it may take the form of genomic DNA, cDNA which has been generated from a mRNA transcript, or DNA generated by nucleic acid amplification. However where the subject DNA does not encode a protein, either genomic DNA or synthetically or recombinantly generated DNA may be the subject of analysis. As would be appreciated by the skilled person, both synthetically and recombinantly generated DNA may also encode all or part of a protein. However, if the subject method is directed to detecting a region of RNA, it would be appreciated that it will first be necessary to reverse transcribe the RNA to DNA, such as using RT-PCR. The subject RNA may be any form of RNA, such as mRNA, primary RNA transcript, ribosomal RNA, transfer RNA, micro RNA or the like. Preferably, said nucleic acid region of interest is a DNA region of interest. To this end, said DNA includes DNA generated by reverse transcription from RNA which is ultimately the subject of analysis, and DNA generated by a nucleic acid amplification method such as PCR.

The present invention therefore more preferably provides a method of amplifying a DNA region of interest as described above.

Reference to "DNA" should be understood as a reference to deoxyribonucleic acid or derivative or analogue thereof. In this regard, it should be understood to encompass all forms of DNA, including cDNA and genomic DNA. The nucleic acid molecules for use in the method of the present invention may be of any origin including naturally occurring (such as would be derived from a biological sample), recombinantly produced or synthetically produced.

Reference to "derivatives" should be understood to include reference to fragments, homologs or orthologs of said DNA from natural, synthetic or recombinant sources. "Functional derivatives" should be understood as derivatives which exhibit any one or more of the functional activities of DNA. The derivatives of said DNA sequences include fragments having particular regions of the DNA molecule fused to other proteinaceous or non-proteinaceous molecules. "Analogs" contemplated herein include, but are not limited to, modifications to the nucleotide or nucleic acid molecule such as modifications to its chemical makeup or overall conformation. This includes, for example, incorporation of novel or modified purine or pyrimidine bases or modification to the manner in which nucleotides or nucleic acid molecules interact with other nucleotides or nucleic acid molecules such as at the level of backbone formation or complementary base pair hybridisation. The biotinylation or other form of labelling of a nucleotide or nucleic acid molecules is an example of a "functional derivative" as herein defined.

Preferably, said DNA is a gene or gene fragment, a chromosomal gene translocation breakpoint or DNA produced by prior nucleic acid amplification.

According to this aspect, the present invention provides a method of amplifying a gene or gene fragment of interest, as described above.

In another aspect, there is provided a method of amplifying a chromosomal gene translocation breakpoint region of interest as described above.

In yet another aspect, there is provided a method of amplifying DNA produced by prior nucleic acid amplification, said method comprising:
(i) contacting a DNA sample with:
   (a) one or more forward primers directed to said DNA produced by prior nucleic acid amplification; and
   (b) one or more reverse primers directed to said DNA produced by prior nucleic acid amplification
   wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the DNA sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified DNA of step (ii) with a third primer which is directed to part or all of the sequence which is complementary to that of the oligonucleotide tag of step (i), wherein said third primer is efficient in the amplification of step (iv); and
(iv) amplifying the DNA sample of step (iii) using the third primer of step (iii) and the efficient primers of group (a) or group (b) of step (i) and (ii), wherein said third primer and efficient primers of group (a) or group (b) hybridise and extend efficiently.

Preferably, said prior nucleic acid amplification is PCR.

In a classical PCR, the primers and reaction conditions are designed so that primer hybridisation and extension of the forward and reverse primers occur at or close to the maximum efficiency so that the number of amplicons approximately doubles with each cycle resulting in efficient exponential amplification. The method of the present invention, however, is predicated on the use of forward and reverse primer sets where the primers of one set have been designed or are otherwise used under conditions wherein they do not hybridise and extend efficiently. Accordingly, although the efficient primer set will amplify normally, the inefficient set will not. As a consequence, when a sequence of interest is amplified, the number of amplicon strands is significantly less than that which would occur in a classical PCR. Efficient amplification only commences once amplicons have been generated which incorporate, at one end, the tag region of the inefficient primer (generation of these amplicons is illustrated in Figure 1 and the functioning of the tag is discussed in more detail hereinafter). At this point, the primers directed to the tag regions effect a normal amplification rate. A "bottleneck" is therefore effectively created in terms of the generation of transcripts from the inefficient primer set.

A more severe bottleneck is usefully created where the primers which are rendered inefficient are degenerate and hybridise widely, or are directed to commonly repeated sequences, such as an *alu* sequence. Amplification of unwanted product may result if the primer binding sites are closely apposed and if the inefficient primers can act as forward primers and reverse primers. However, owing to both primers being inefficient, amplification is initially extremely inefficient and there is a severe bottleneck. This situation is illustrated in Figure 2. Efficient amplification only commences once amplicon strands have been generated which comprise the tag region of the inefficient primer at one end and its complement at the other. After any given number of cycles, the number of such amplicons is, however, substantially less than that which occurs during amplification of the sequence of interest, as described above. The amount of unwanted product at the end of the amplification reaction is correspondingly reduced.

Hybridisation and extension of an inefficient primer which has correctly hybridised to the sequence of interest followed in a subsequent cycle by hybridisation and extension of an efficient primer to the previously synthesised amplicon generates a template to which the tag primer can efficiently hybridise and extend. Since such molecules together with their complements provide upstream and downstream binding sites, each for an efficient primer (the tag primer and one member of the efficient set), succeeding cycles of amplification from such templates are both efficient and exponential. The result is that, after an initial lag or "bottleneck", the overall rate of amplification speeds up in later cycles so that a near doubling of amplicon number with each cycle results. However, the net result is that there is negative selection against amplification of undesired amplicons as compared to amplicons of the sequence of interest, owing to the bottleneck at each end for the former and only at one end for the latter.

Accordingly, if the same number of commencing target sequences is considered and comparison to the amplification produced by classical PCR is made, application of the method of the present invention will produce a lesser increase in the number of amplicons of the sequence of interest and an even lesser increase in the number of amplicons of unwanted sequences, as illustrated in Figure 8. Although amplification of both wanted and unwanted products occurs, there is relative enrichment of the sequence of interest relative to the unwanted sequences. There is an inverse relationship between absolute amplification and enrichment since decreasing the efficiency of the inefficient primer set produces increased enrichment at the expense of lesser amplification.

Those skilled in the art will appreciate that the amplifications of the first and second phases of the method do not need to be performed as physically separate reactions but can be simply and conveniently performed in the same reaction container; the first phase commences with cycle 1 and proceeds thereafter whereas the second phase commences at cycle 3 and proceeds thereafter.

In terms of deciding whether it should be the forward primer set or the reverse primer set which is rendered inefficient, this may vary from one situation to the next. In general, however, it is likely that one would seek to render inefficient the hybridisation and extension of the primer set which exhibits the greatest probability of binding to sequences other than the target of interest. In terms of the exemplification provided herein, the experimental design for the enrichment of the *BCR-ABL* breakpoint is based on rendering inefficient the *ABL* primer set rather than the *BCR* primer set. In yet another example, if the primer target sequence of a DNA region of interest is known on only one side of the DNA region, a universal or degenerate primer could be used to initiate primer extension and amplification from the other side. However, since such a primer would, by definition, bind promiscuously across the sample DNA, by rendering this primer set inefficient, an enhanced amplification of the DNA region of interest compared to that of irrelevant sequences can be achieved. In still another example, in terms of deciding whether it should be the forward primer set or the reverse primer set which is rendered inefficient when the DNA sample being amplified has been produced by prior nucleic acid amplification, one might seek to render inefficient the hybridisation and extension of the forward primer(s) if the forward primer(s) in the initial commencing amplification were promiscuous or, conversely, the hybridisation and extension of the reverse primer(s) if the reverse primer(s) in the initial commencing amplification were promiscuous.

Preferably, the primer group which is rendered inefficient is the group which has the potential to hybridise promiscuously to non-target DNA regions.

Reference to a "primer" or an "oligonucleotide primer" should be understood as a reference to any molecule comprising a sequence of nucleotides, or functional derivatives or analogues thereof, the function of which includes hybridisation to a region of a nucleic acid molecule of interest (the DNA of interest interchangeably being referred to as a "target DNA"). It should be understood that the primer may comprise non-nucleic acid components. For example, the primer may also comprise a non-nucleic acid tag such as a fluorescent or enzymatic tag or some other non-nucleic acid component which facilitates the use of the molecule as a probe or which otherwise facilitates its detection or immobilisation. The primer may also comprise additional nucleic acid components, such as the oligonucleotide tag which is discussed in more detail hereinafter. In another example, the primer may be a protein nucleic acid which comprises a peptide backbone exhibiting nucleic acid side chains. Preferably, said oligonucleotide primer is a DNA primer.

Reference to "forward primer" should be understood as a reference to a primer which amplifies the target DNA in the DNA sample of interest by hybridising to the antisense strand of the target DNA.

Reference to "reverse primer" should be understood as a reference to a primer which amplifies the target DNA in the DNA sample of interest and in the PCR by hybridising to the sense strand of the target DNA.

The design and synthesis of primers suitable for use in the present invention would be well known to those of skill in the art. In one embodiment, the subject primer is 4 to 60 nucleotides in length, in another embodiment 10 to 50 in length, in yet another embodiment 15 to 45 in length, in still another embodiment 20 to 40 in length, in yet another embodiment 25 to 35 in length. In yet still another embodiment, primer is about 26, 27, 28, 29, 30, 31, 32, 33 or 34 nucleotides in length.

In terms of the number of primers which are used in the method of the invention, this can be determined by the person of skill in the art. If the sequences of the two ends of the sequence of interest are known then only one forward and one reverse primer may be needed, but if this information is not available then multiple forward and/or reverse primers or one or more degenerate primers may be employed. For isolation of translocation breakpoints, which may occur at unknown points within large regions of the interacting genes, multiple primers may be used in an attempt to ensure that at least 1 forward and 1 reverse primer closely span the breakpoint so that efficient PCR amplification will occur. With regard to chronic myeloid leukaemia (CML), for example, nearly all *BCR* translocations involve one of two regions, each of approximately 3 kb in length. In this case, 12 outer forward primers may be used. The *ABL* gene, however, is larger, the region commonly involved in translocation is approximately 140 kb in length, and up to 282 or more outer reverse primers may be used. In one particular embodiment, a combination of 6 forward primers and 24 reverse primers is used and in another embodiment a combination of 1 forward primer and 282 reverse primers. In yet another combination there is 6 forward primers and 270-310 reverse primers. In yet another combination there is 1-20 forward primers and 250-350 reverse primers. The primer number which is selected to be used will depend on the target region of interest and thus may vary from one target to the next. As would be understood by the person of skill in the art, in terms of classical PCR a large number of primers in each individual PCR reaction increases the probability of non-specific amplification reactions. The method of the present invention, however, enables the use of a larger number of primers due to the minimisation of non-specific amplification reactions by virtue of rendering one primer set functionally inefficient.

It should also be understood that to the extent that there are 2 or more different primers within a single forward or reverse primer pool, one can render all the primers within that pool inefficient or one can render inefficient only a select subgroup of these primers which are thought to be the most problematic in terms of generating irrelevant amplicons.

By "functionally inefficient" is meant that the subject primer has been modified and/or is utilised under environmental conditions which render its hybridisation and extension less effective in terms of the number and/or rate of amplicon generation from that primer than if the design of the primer had not been modified or it had not been used under the subject environmental conditions. Methods of rendering a primer functionally inefficient would be well known to the person of skill in the art and include but are not limited to:
(i) reducing the concentration of primer which is used;
(ii) reducing the hybridisation time of the primer;
(iii) increasing the temperature at which the hybridisation reaction is required to occur;
(iv) modifying the length of the primer;
(v) altering the primer melting temperature;
(vi) introducing a chaotropic agent during the hybridisation/primer extension phase;
(vii) introducing into the reaction a competitive inhibitor to the primer, such as its complementary sequence;
(viii) introducing nucleotide mismatches into the primer sequence;
(ix) using primer analogs which hydrogen-bond less efficiently in the context of hybridisation.

It would be appreciated that in addition to potentially modifying the primer itself, one can alternatively (or additionally) elect to modify the reaction conditions to achieve the same outcome. To this end, it should be appreciated that one could also design a system which uses two or more of the above-listed methods to achieve the functional inefficiency of one primer without similarly rendering inefficient the other primer. This is more likely to become an issue where one elects to modify the reaction conditions rather than the primer itself. For example, if one elects to increase reaction temperature in order to reduce efficiency, this will affect the functionality of both the primer groups (i.e. the forward primers and the reverse primers). Accordingly, to minimise how far the temperature is required to be increased, one may combine this with the use of a primer which has been increased in length in order to maximise the inefficiency of one primer but not the other. In yet another example, one may choose not to alter reaction conditions but, instead, may reduce the concentration of primer which is utilised. In another example, one may reduce the length of the primer or reduce its concentration in combination with a reduced hybridisation time. Designing and altering these factors to achieve functional inefficiency would be well known to the person of skill in the art since they are issues which are routinely considered and well described in the art in the context of designing PCR reaction (Sambrook J. and Russell D. "Molecular cloning: a laboratory manual" 3rd edition published by Cold Spring Harbor, 2001), albeit usually in the context of reducing the possibility of the functional inefficiency of the forward and reverse primers as opposed to deliberately inducing this state. Nevertheless, the considerations which are required to be made in order to design an efficient PCR reaction are the same as the ones which are made to design part of the reaction to function inefficiently. Accordingly, the issue is merely one of how these considerations will be applied. To this end, it should also be appreciated that the notion of rendering a primer functionally inefficient encompasses not just modifying the design of the primer itself but also or alternatively modifying the reaction conditions within which the primer is required to function.

Preferably, the functional inefficiency is hybridisation inefficiency which is achieved by one or more of modifying primer length, sequence, annealing temperature, starting concentration or hybridisation time.

Preferably, said functionally inefficient primer group is the primer group which, in the absence of having been rendered functionally inefficient has the potential to hybridise promiscuously to non-target DNA regions.

In another preferred embodiment, said DNA region of interest is a gene or gene fragment or a chromosomal gene translocation breakpoint.

As detailed hereinbefore, the method of the present invention is aimed at enriching a sequence of interest and is predicated on rendering either the forward or reverse primer group inefficient. As a consequence, amplification of the sequence of interest is inefficient, but it is much more inefficient for other unwanted sequences which are amplified by one or more of the members of the inefficient primer group acting as both forward and reverse primers. This situation, which is exemplified in Figure 2, results in negative selection against such unwanted sequences and enrichment of the sequence of interest. However, it is also desirable to revert to efficient levels of amplification in order to facilitate increasing the copy number of the amplicon of interest. This is conveniently achieved by incorporating into the functionally inefficient primer a tag which can itself function to generate a primer hybridisation target site. By this means there is effectively enabled the ability to effect efficient amplification from the same direction as the functionally inefficient primer.

The generation of such templates, which enable efficient priming by the tag primer in later cycles, results in further enrichment, and the sequence of events is shown in Figure 1. Primer 2, which comprises an oligonucleotide tag, hybridises to a template strand, which is either an original genomic strand or an amplicon strand generated in a previous PCR cycle. This primer hybridises inefficiently but it does nevertheless generate some complementary strands during this first cycle. In a second cycle of amplification, primer 1 hybridises to the primer 2 generated amplicon strands and extends, generating a strand comprising a binding site for the tag primer, which is able to be efficiently amplified by the tag primer in later cycles. The Figure illustrates the situation where primer 1 is an efficient primer which selects for those amplicons which correspond to the DNA region of interest and which efficiently generates amplicon strands containing a binding site for the tag primer. For situations characterised by forward and reverse priming by one or more members of the group of inefficient primers, such as amplification of common sequences such as Alu or sequences able to be amplified by a degenerate or universal primer, and for which amplification is undesired, the primer corresponding to primer 1 in Figure 1 is inefficient and its extension in the second cycle generates fewer strands containing the binding site for the tag primer. Accordingly, there effectively occurs a further negative selection against production of such undesired templates and a positive selection in favour of template strands of the sequence of interest, which comprise primer 1 at one end, and the binding site for the tag at the other end.

In order to ensure that these oligonucleotide tags do not interfere with the extension of the primer, the primers are linked to the oligonucleotide tag at their 5' end. Reference to "oligonucleotide tag" should therefore be understood as a reference to a nucleotide sequence of usually less than 50 nucleotides which is linked to the 5' end of the functionally inefficient primer for use in the method of the present invention. In one embodiment, the tag is 25-30 bases in length. It should also be understood that consistently with the definitions provided in relation to the forward and reverse primers, the oligonucleotide tags herein described may also comprise non-nucleic acid components such as isolation or visualisation tags eg. biotin, enzymatic labels, fluorescent labels and the like. This enables quick and simple isolation or visualisation of the tagged primers or amplicons via non-molecular methods.

That the oligonucleotide tag is "operably linked" to the primer should be understood as a reference to those regions being linked such that the functional objectives of the tagged primer, as detailed hereinbefore, can be achieved. In terms of the means by which these regions are linked and, further, the means by which the subject oligonucleotide primer binds to its target DNA region, these correspond to various types of interactions. In this regard, reference to "interaction" should be understood as a reference to any form of interaction such as hybridisation between complementary nucleotide base pairs or some other form of interaction such as the formation of bonds between any nucleic or non-nucleic acid portion of the primer molecule or tag molecule with any other nucleic acid or non-nucleic acid molecule, such as the target molecule, a visualisation means, an isolation means or the like. This type of interaction may occur via the formation of bonds such as, but not limited to, covalent bonds, hydrogen bonds, van der Wals forces or any other mechanism of interaction. Preferably, to the extent that the interaction occurs between the primer and a region of the target DNA, said interaction is hybridisation between complementary nucleotide base pairs. In order to facilitate this interaction, it is preferable that the target DNA is rendered partially or fully single stranded for a time and under conditions sufficient for hybridisation with the primer to occur.

It should be understood that the oligonucleotide primers and tags for use in the method of the present invention should not be limited to the specific structure exemplified herein (being a linear, single-stranded molecule) but may extend to any suitable structural configuration which achieves the functional objectives detailed herein. For example, it may be desirable that all or part of the oligonucleotide is double stranded, comprises a looped region (such as a hairpin bend) or takes the form of an open circle confirmation, that is, where the nucleotide primer is substantially circular in shape but its terminal regions do not connect.

Facilitating the interaction of the nucleic acid primer with the target DNA may be performed by any suitable method. Those methods will be known to those skilled in the art. To this end, it should be understood that the primer directed to the tag can be incorporated into the reaction tube at any suitable time point. For example, it may be incorporated prior to the commencement of the initial amplification cycles, that is together with the forward and reverse primer sets, or it may be incorporated subsequently to the initial two amplification cycles. In either case, the primer to the tag region will become functional only after amplicons have been generated which incorporate the tag region, as hereinbefore described.

Methods for achieving primer directed amplification are also very well known to those of skill in the art. In a preferred method, said amplification is polymerase chain reaction, NASBA or strand displacement amplification. Most preferably, said amplification is polymerase chain reaction.

Methods for performing serial nucleic acid amplification, utilising product from a prior amplification as template for a subsequent reaction, are also very well known to those of skill in the art. In another preferred method, as performed for example to produce the results shown in Figures 3-12, a bottleneck amplification may be performed to produce selective amplification of a DNA sequence of interest which has been produced by either a prior bottleneck PCR or a prior non-selective PCR. Those with skill in the art will appreciate that sequential bottleneck PCRs can be performed.

Preferably, said DNA region of interest has been produced by prior nucleic acid amplification.

In another preferred embodiment, said DNA region of interest is a gene or gene fragment or a chromosomal gene translocation breakpoint such as the *BCR-ABL* breakpoint.

In terms of effecting the method of the present invention, it should be appreciated that the primers of steps (i) and (iii) can be simultaneously added to the reaction solution prior to the first two amplification cycles, or the primers of step (iii) can be introduced after the first or second cycle of amplification. This will depend on how the skilled person is seeking to perform the PCR reaction. For example, for ease of use, it is often desirable to be able to perform the entire reaction in a single tube. Nevertheless, any other method of achieving the steps of the invention can be used.

Reference to a "sample" should be understood as a reference to either a biological or a non-biological sample. Examples of non-biological samples includes, for example, the nucleic acid products of synthetically produced nucleic acid populations. Reference to a "biological sample" should be understood as a reference to any sample of biological material derived from an animal, plant or microorganism (including cultures of microorganisms) such as, but not limited to, cellular material, blood, mucus, faeces, urine, tissue biopsy specimens, fluid which has been introduced into the body of an animal and subsequently removed (such as, for example, the saline solution extracted from the lung following lung lavage or the solution retrieved from an enema wash), plant material or plant propagation material such as seeds or flowers or a microorganism colony. The biological sample which is tested according to the method of the present invention may be tested directly or may require some form of treatment prior to testing. For example, a biopsy sample may require homogenisation prior to testing or it may require sectioning for *in situ* testing. Further, to the extent that the biological sample is not in liquid form, (if such form is required for testing) it may require the addition of a reagent, such as a buffer, to mobilise the sample.

To the extent that the target DNA is present in a biological sample, the biological sample may be directly tested or else all or some of the nucleic acid material present in the biological sample may be isolated prior to testing. It is within the scope of the present invention for the target nucleic acid molecule to be pre-treated prior to testing, for example inactivation of live virus or being run on a gel. It should also be understood that the biological sample may be freshly harvested or it may have been stored (for example by freezing) prior to testing or otherwise treated prior to testing (such as by undergoing culturing).

Reference to "contacting" the sample with the primer should be understood as a reference to facilitating the mixing of the primer with the sample such that interaction (for example, hybridisation) can occur. Means of achieving this objective would be well known to those of skill in the art.

The choice of what type of sample is most suitable for testing in accordance with the method disclosed herein will be dependent on the nature of the situation, such as the nature of the condition being monitored. For example, in a preferred embodiment a neoplastic condition is the subject of analysis. If the neoplastic condition is a leukaemia, a blood sample, lymph fluid sample or bone marrow aspirate would likely provide a suitable testing sample. Where the neoplastic condition is a lymphoma, a lymph node biopsy or a blood or marrow sample would likely provide a suitable source of tissue for testing. Consideration would also be required as to whether one is monitoring the original source of the neoplastic cells or whether the presence of metastases or other forms of spreading of the neoplasia from the point of origin is to be monitored. In this regard, it may be desirable to harvest and test a number of different samples from any one mammal. Choosing an appropriate sample for any given detection scenario would fall within the skills of the person of ordinary skill in the art.

The term "mammal" to the extent that it is used herein includes humans, primates, livestock animals (e.g. horses, cattle, sheep, pigs, donkeys), laboratory test animals (e.g. mice, rats, rabbits, guinea pigs), companion animals (eg. dogs, cats) and captive wild animals (eg. kangaroos, deer, foxes). Preferably, the mammal is a human or a laboratory test animal. Even more preferably the mammal is a human.

As detailed hereinbefore, the method of the present invention is preferably performed as a sequential series of amplification cycles. To this end, a minimum of two cycles of amplification is required at step (ii) in order to effect the generation of amplicons which are suitable to undergo amplification by the primer to the oligonucleotide tag of the inefficient primer and the original primer which retained efficiency. It should be understood, however, that the method of the invention may be designed to conduct 3 or more amplification cycles before contact with the primer to the oligonucleotide tag is effected. Alternatively, if all the reagents of the present method are introduced to the reaction tube prior to commencement, more than two cycles of amplification before the tag-based amplification of step (iv) becomes effective may not occur. It should also be understood that, even within the same PCR, each amplification cycle may generate new amplicons which are then amenable to subsequent tag-based amplification.

Although the method of the present invention has been designed such that the rounds of amplification can be sequentially performed directly on the amplification product of a previous round of amplification, this should not be understood as a limitation in terms of whether any additional steps are sought to be incorporated by the skilled person, such as enrichment/selection steps. For example, one may seek to select or enrich for the desired amplicons after the first round of amplification and to thereafter conduct the second round of amplification on that material alone. Methods which one could utilise to select or enrich include:
(i) a selection step based on the unique markers which are linked to the primers. For example, biotinylation of one of the primers provides means of identifying and isolating amplicons which have resulted from extension by either the forward or reverse primers. By flooding the amplification product with biotinylated primer, the primer can act as a probe to identify the amplicons of interest and the biotinylation can provide a basis for isolating those amplicons. By ensuring that each of the primer groups for use in the method of the present invention comprises a unique tag, it is possible to select out, with significant particularity, only specific amplicons of interest. In particular, the skilled person would seek to exclude amplicons which have been amplified by a forward primer but which have not then been amplified by a reverse primer.
(ii) running the products on a gel and excising out only certain bands or regions which are likely to be relevant and thereafter subjecting these to a further amplification step. When a band is present on the gel after the second cycle amplification, if there are any problems in sequencing an attempt can be made to clean it up by cutting the product out of the gel and performing a series of PCR reactions using individual primers and/or smaller pools of primers.

Although the method of the present invention may be adapted to include any such additional steps, one of the unique advantages of the present method is that it has been designed in order to minimise the generation of irrelevant amplicons, thereby minimising the need to implement enrichment or selection steps. Nevertheless, depending on the particular situation, the incorporation of such steps may nevertheless be useful.

In another example, one may wish to adapt the current method to combine in various ways one or more amplifications using the current method with one or more other amplification steps in order to increase specificity and facilitate isolation of the desired product. Figures 6 and 11 illustrate the results of experiments in which 1 standard PCR was followed by 3 rounds of bottleneck PCR. Figure 7 illustrates the results of an experiment in which 2 rounds of standard PCR were followed by 2 cycles of bottleneck PCR. Figures 9, 10 and 12 illustrate the results of experiments in which 1 standard PCR was followed by 2 rounds of bottleneck PCR. It is also possible to alternate cycles of standard and bottleneck PCR. Accordingly, one can optimise the amplification of the sequences of interest by any suitable means such as increasing the number of PCR cycles in the second phase of the method or by performing a subsequent PCR or other form of amplification. The method can also be applied repeatedly in order to provide further enrichment and amplification of the sequence of interest, to the degree desired. Examples showing progressive enrichment of a sequence of interest by repeated application of the method are shown in Figures 5 and 11; calculation using the data suggest that the efficiency of the inefficient primers used in rounds 2 and 3 of these experiments was approximately 1% of maximum. These various experimental designs will be understood by those of skill in the art.

The provision of an efficient means of amplifying a nucleic acid region of interest is useful in a range of applications including, but not limited to, the diagnosis and/or monitoring of disease conditions which are characterised by specific gene sequences, the characterisation or analysis of gene regions of interest, the identification or characterisation of DNA breakpoint regions and the isolation of gene sequences of interest where only the nucleotide sequence at one end of the gene sequence of interest is either known or can be inferred.

The present invention is further described by reference to the following non-limiting examples.

### EXAMPLE 1

### BCR-ABL DNA breakpoint amplification using Bottleneck PCR

*BCR-ABL* DNA breakpoints were amplified using *BCR* and *ABL*-specific primers in a four round PCR screen. Six *BCR*-specific primers and 282 *ABL*-specific primers were designed spanning the major breakpoint regions of *BCR* (3.2kb) and *ABL* (140kb) DNA respectively.

The first round PCR amplifications were performed in 25µls containing 50ng of a single *BCR*-specific primer, 100ng of all 282*ABL*-specific primers (350pg of each primer), 50ng of Tag A, 50ng genomic DNA, 50mM KCl, 2mM Tris HCl (pH 8.4), 1U Platinum Taq DNA polymerase (Invitrogen), 5mM MgCl₂ and 300µm of each of dUTP, dATP, dGTP and dTTP. The amplification conditions were: 95° for 4 minutes; then 6 cycles with 97° for 1 minute, 65° for 1 minute with the temperature decreasing 1° every 2 cycles, 72° for 1 minute; then 4 cycles with 96° for 30 seconds, 62° for 1 minute with the temperature decreasing 1° after the first 2 cycles, 72° for 1 minute; then 10 cycles with 94° for 30 secs, 61° for 1 minute, 72° for 1 minute.

The *ABL*-specific primers have a Tag region (Tag A) at the 5' end of the primer that is not complementary to *ABL* DNA. In the first round of PCR the Tag sequence attached to the *ABL*-specific primers is incorporated into amplicons, enabling the DNA to be further amplified in subsequent rounds of PCR using the *BCR* primer together with the Tag A primer rather than the *ABL*-specific reverse primers. Each round of PCR uses different Tag sequences.

The second, third and fourth round PCR amplifications were performed in 25µls containing a dilution of the previous PCR round reaction mix (at a dilution factor of 100), 50ng of a single *BCR*-specific nested primer, 500pg chimaeric Tag primers (Tag A/I, I/1, 1/2 in the second, third and fourth rounds respectively), 50ng of a single Tag primer (Tag I, 1, 2 in the second, third and fourth rounds respectively), 50mM KCl, 2mM Tris HCl (pH 8.4), 1U Platinum Taq DNA polymerase (Invitrogen), 5mM MgCl₂ and 300µm of each of dUTP, dATP, dGTP and dTTP. The amplification conditions were: 95° for 4 minutes; then 20 cycles with 94° for 30 seconds, 65° for 1 minute, 72° for 1 minute.

### EXAMPLE 2

### PML-RARα DNA breakpoint amplification using bottleneck PCR

Bottleneck PCR was used to isolate the *PML-RARα* translocation breakpoint from a patient with acute promyelocytic leukemia. The patient DNA was amplified using multiple RARα primers and a single PML primer and then 2 rounds of bottleneck PCR were performed. The amplified DNA electrophoresed on a 2% agarose gel (Figure 9a). To confirm that the breakpoint had been isolated, the breakpoint sequence was used to design RARα and PML primers spanning the breakpoint, and the patient DNA was amplified for one round using these primers (Figure 9b). The sequence of the amplified band shown on the gel in Figure 9a was also determined (Figure 9c).

### EXAMPLE 3

### Gene walking using a degenerate primer and bottleneck PCR

Gene walking along three genes, *APC, BRCA1* and myocillin, was performed using 50 ng of a gene-specific forward primer, 50 ng of one of a variety of degenerate reverse primers, and 50 ng of a reverse tag primer. The degenerate reverse primers had 4-6 random normal residues at the 3' end, followed by 3-6 degenerate residues, followed by a random tag sequence of 12-18, usually 18, normal residues. The most commonly used degenerate primer had 5 fixed bases at the 3' end followed by 5 degenerate bases, followed by a tag sequence of 18 bases (5'TGCTAGGATCCAAGGNNNNNATTCG3' (SEQ ID NO:1)). The reverse tag primer had the same sequence as the tag on the degenerate reverse primer. Five PCR cycles, with annealing at 35° C. for five minutes, were followed by 15 cycles with annealing at 55° C for 3 minutes.

PCR was performed in a 25µl volume, with 50ng total DNA, 5mM MgCl₂, 0.1mM dUTP, 0.2mM dTTP, and 0.3 mM of each of dCTP, dATP and dGTP, and 1 unit of Platinum Taq polymerase (Invitrogen). Primers were from Sigma-Aldrich (St. Louis, MO, USA) or Invitrogen (Carlsbad, CA, USA). PCR cycling conditions typically involved denaturation at 94° C. for 30 seconds, annealing as described above, and extension at 72° C. for 90 seconds.

Between 1 and 3 rounds of bottleneck PCR were performed, usually 2. A 1/100 dilution of the amplified material from the primary round described above was amplified in the first bottleneck PCR and a 1/1000 dilution of the amplified material from the previous round was used for each subsequent round. Each PCR round was run for 20 cycles except when electrophoresis was to be performed, in which case it was run to completion, for 30 - 40 cycles. Each PCR contained 50 ng of a nested forward primer, 0.5 ng of a hybrid reverse primer and 50 ng of a tag reverse primer. The hybrid reverse primer consisted of a 3' end, which had the same sequence as the tag primer for the previous PCR round, and a 5' end, which had a new tag sequence. The tag reverse primer had the same sequence as the new tag sequence.

PCR products were examined by gel electrophoresis and discrete bands were isolated and sequenced usually with the forward primer and the tag primer used for the final amplification. In some experiments involving gene walking, the entire amplified product was sequenced, irrespective of whether 1 or more discrete bands had been visualised. In one experiment involving gene walking, the sequencing reaction was performed using primers 732 bp and 1302 bp downstream from the initial sequencing primer.

### EXAMPLE 4

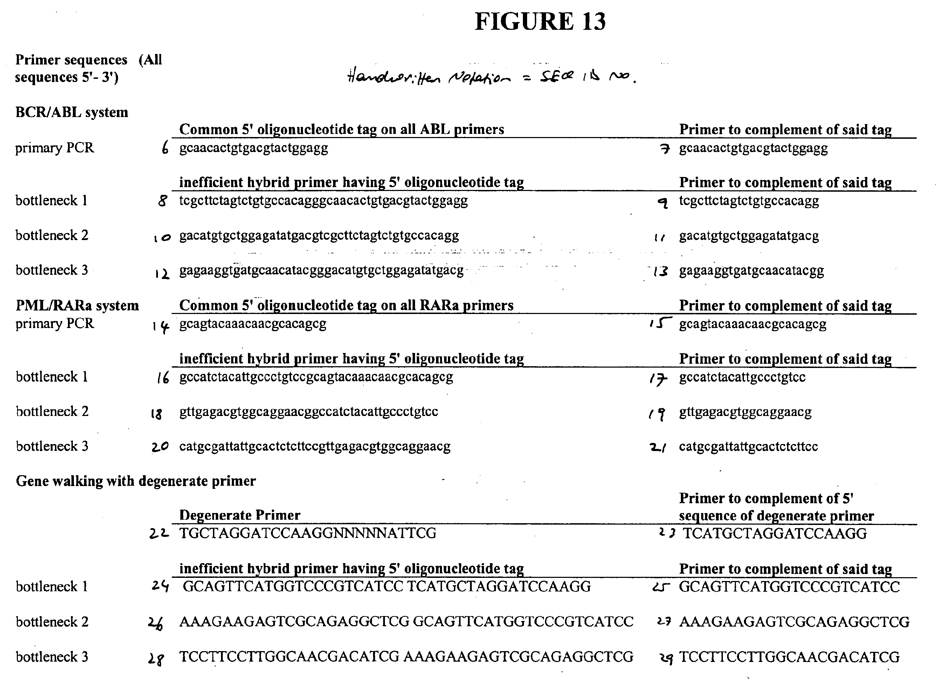

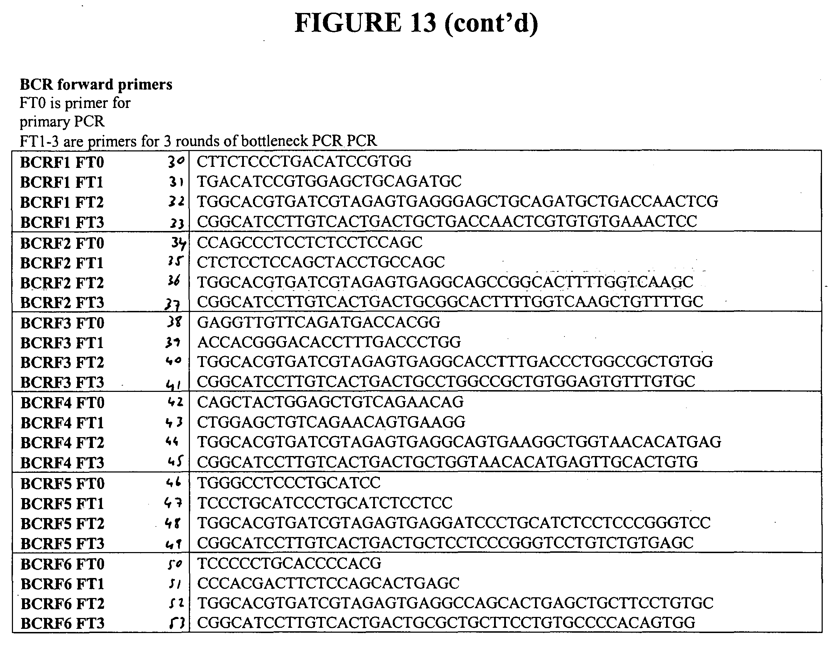

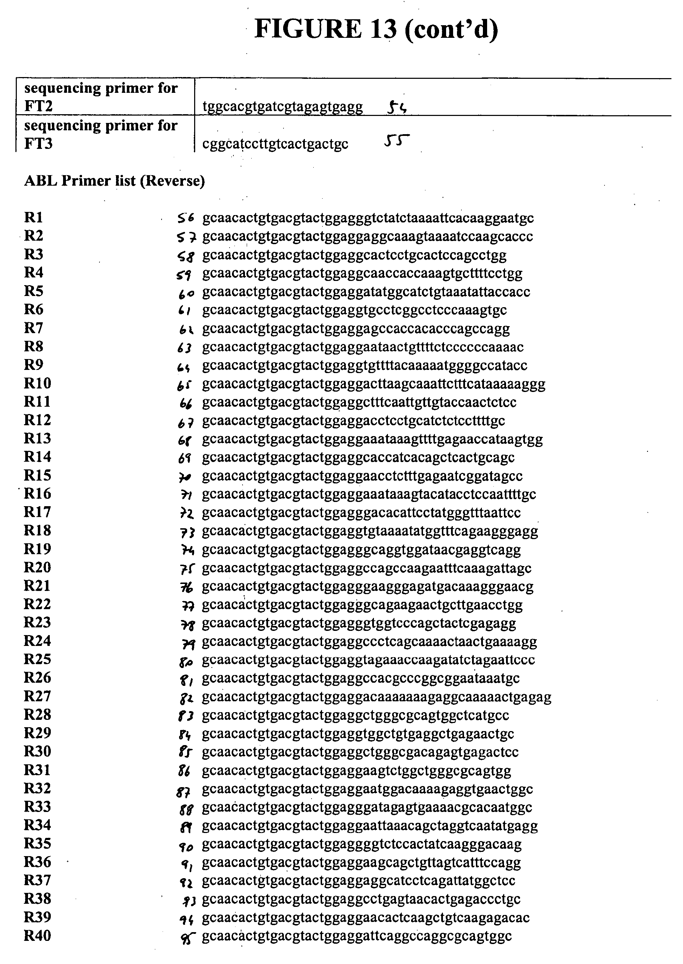

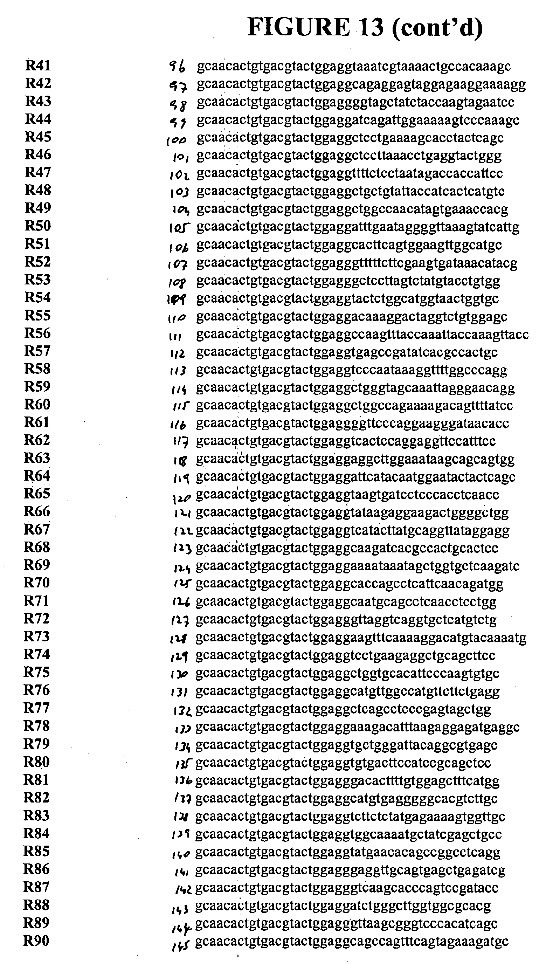

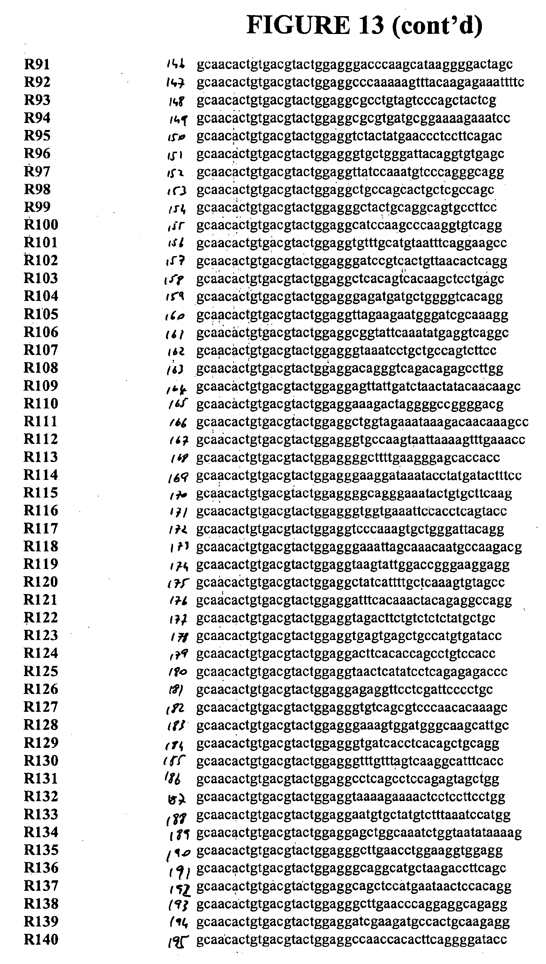

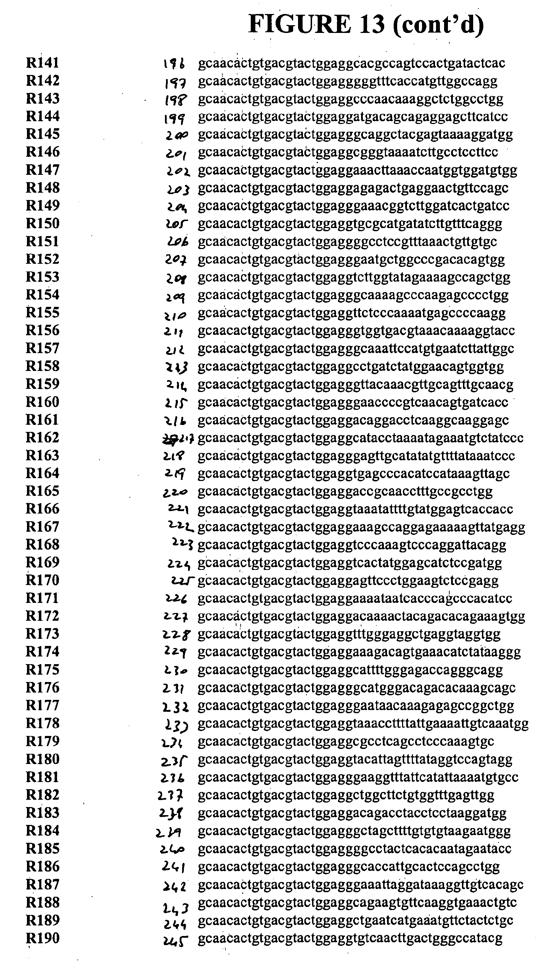

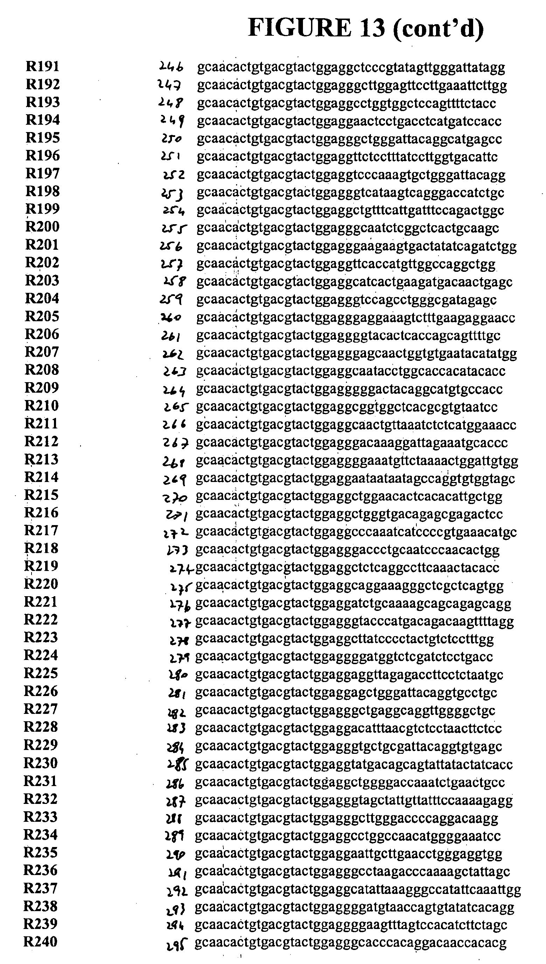

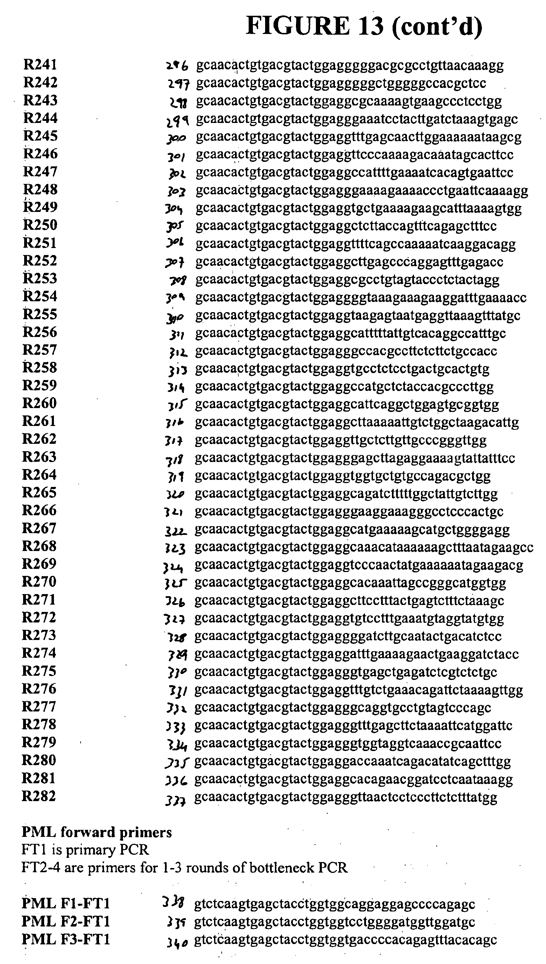

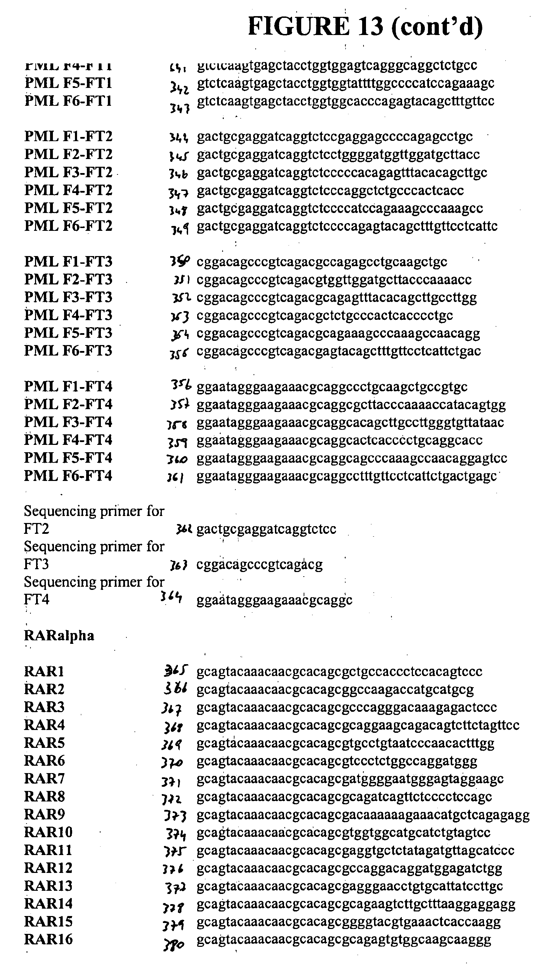

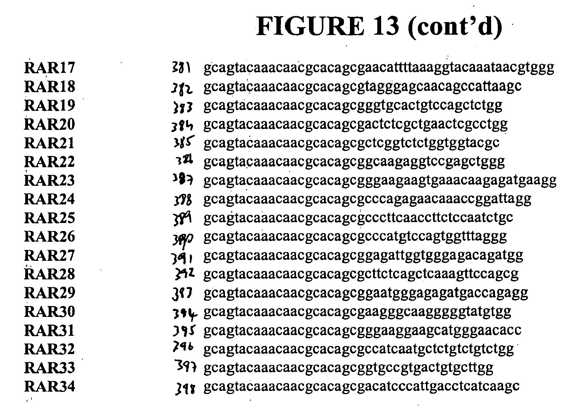

Figure 13 provides examples of primers and tag sequences suitable for use in the method of the present invention.

### BIBLIOGRAPHY

Cheng et al., 1994, Effective amplification of long targets from cloned inserts and human genomic DNA. Proc Natl Acad Sci. 91:5695-5699
Sambrook and Russel, 2001, Molecular Cloning: A Laboratory Manual, 3rd Ed. Cold Spring Harbor, N.W.: Cold Spring Harbor Laboratory Press. Chapter 8: In vitro Amplification of DNA by the Polymerase Chain Reaction.
Nailis H, Coenye T, Van Nieuwerburgh F, Deforce D, Nelis HJ (2006). "Development and evaluation of different normalization strategies for gene expression studies in Candida albicans biofilms by real-time PCR". BMC Mol Biol. 7: 25
Nolan T, Hands RE, Bustin SA (2006). "Quantification of mRNA using real-time RT-PCR". Nat. Protoc. 1: 1559-1582

## Claims

1. A method of amplifying a nucleic acid region of interest, said method comprising:
(i) contacting a nucleic acid sample with:
(a) one or more forward primers directed to said region of interest; and
(b) one or more reverse primers directed to said region of interest
wherein the primers of one of group (a) or group (b) are functionally inefficient in the amplification reaction of step (ii) and are operably linked at their 5' end to an oligonucleotide tag and wherein the primers of the other of group (a) or group (b) are efficient in the amplification of step (ii);
(ii) amplifying the nucleic acid sample of step (i) through at least two cycles of amplification using primers (a) and (b) wherein the tagged primers of (a) or (b) hybridise and extend inefficiently and the other primers of (a) or (b) hybridise and extend efficiently;
(iii) contacting the amplified nucleic acid of step (ii) with a third primer which is directed to part or all of the sequence which is complementary to that of the oligonucleotide tag of step (i), wherein said third primer is efficient in the amplification of step (iv); and
(iv) amplifying the nucleic acid sample of step (iii) using the third primer of step (iii) and the efficient primers of group (a) or group (b) of step (i) and (ii), wherein said third primer and efficient primers of group (a) or group (b) hybridise and extend efficiently.

2. The method according to claim 1 wherein said nucleic acid is DNA.

3. The method according to claim 1 or 2 wherein said amplified nucleic acid of step (iv) is isolated and/or analysed.

4. The method according to any one of claims 1 to 3 wherein said nucleic acid region of interest is a region of a DNA molecule which has been generated by an amplification method, preferably wherein said amplification method is PCR.

5. The method according to any one of claims 1 to 4 wherein the amplification methods of steps (ii) and (iv) are PCR, NASBA or strand displacement amplification.

6. The method according to any one of claims 1 to 5 wherein said primer is 4-60 nucleotides in length, 10-50 nucleotides in length, 15-45 nucleotides in length, 20-40 nucleotides in length or 25-35 nucleotides in length, preferably wherein said primer is 26, 27, 28, 29, 30, 31, 32, 33 or 34 nucleotides in length.

7. The method according to any one of claims 1 to 6 wherein said primer is rendered functionally inefficient by:
(i) reducing the concentration of primer which is used;
(ii) reducing the hybridisation time of the primer;
(iii) increasing the temperature at which the hybridisation reaction is required to occur;
(iv) modifying the length of the primer;
(v) altering the primer melting temperature;
(vi) introducing a chaotropic agent during the hybridisation/primer extension phase;
(vii) introducing into the reaction a competitive inhibitor to the primer, such as its complementary sequence;
(viii) introducing nucleotide mismatches into the primer sequence;
(ix) using primer analogs which hydrogen-bond less efficiently in the context of hybridisation.

8. The method according any one of claims 1 to 7 wherein the primer group which is rendered inefficient is the primer group which has the potential to hybridise promiscuously to non-target DNA regions, preferably wherein said primer is a degenerate primer.

9. The method according to any one of claims 1 to 8, wherein said region of interest is a region of genomic DNA, a gene, part of a gene, a DNA recombination product or a chromosomal gene translocation breakpoint.

10. The method according to claim 9 wherein said chromosomal gene translocation breakpoint is BCR-ABL, preferably wherein there is used 1-20 forward primers directed to BCR and 250-350 reverse primers directed to ABL.

11. The method according to claim 9 wherein said chromosomal gene translocation breakpoint is PML-RARα.

12. The method according to claim 10 wherein there is used 12 forward primers directed to *BCR* and 282 reverse primers directed to *ABL.*

13. The method according to claim 10 wherein there is used 6 forward primers directed to *BCR* and 24 reverse primers directed to *ABL.*

14. The method according to claim 10 wherein there is used 1 forward primer directed to *BCR* and 282 reverse primers directed to *ABL.*

15. The method according to claim 10 wherein there is used 6 forward primers directed to BCR and 270-310 reverse primers directed to ABL.

## Patentansprüche

1. Verfahren zum Amplifizieren einer Nukleinsäureregion, die von Interesse ist, wobei das Verfahren Folgendes umfasst:
(i) das Kontaktieren einer Nukleinsäureprobe mit:
(a) einem oder mehreren Vorwärtsprimern, die auf die Region, die von Interesse ist, gerichtet sind; und
(b) einem oder mehreren Rückwärtsprimern, die auf die Region, die von Interesse ist, gerichtet sind
wobei die Primer einer von der Gruppe (a) oder Gruppe (b) funktionell in der Amplifikationsreaktion von Schritt (ii) ineffizient sind und funktionsfähig an ihrem 5'-Ende an eine Oligonukleotid-Markierung angeknüpft sind und wobei die Primer der anderen von der Gruppe (a) oder Gruppe (b) bei der Amplifikation von Schritt (ii) effizient sind;
(ii) das Amplifizieren der Nukleinsäureprobe aus Schritt (i) durch mindestens zwei Amplifikationszyklen unter Anwendung von Primern (a) und (b), wobei die markierten Primer von (a) und (b) hybridisieren und sich effizient verlängern und die anderen Primer von (a) oder (b) hybridisieren und sich effizient verlängern;
(iii) das Kontaktieren der amplifizierten Nukleinsäure aus Schritt (ii) mit einem dritten Primer, der auf einen Teil oder die gesamte Sequenz gerichtet ist, die der Oligonukleotidmarkierung aus Schritt (i) komplementär ist, wobei der dritte Primer bei der Amplifikation von Schritt (iv) effizient ist; und
(iv) das Amplifizieren der Nukleinsäureprobe aus Schritt (iii) unter Anwendung des dritten Primers aus Schritt (iii) und der effizienten Primer der Gruppe (a) oder Gruppe (b) aus Schritt (i) und (ii), wobei der dritte Primer und die effizienten Primer der Gruppe (a) oder Gruppe (b) hybridisieren und sich effizient verlängern.

2. Verfahren nach Anspruch 1, wobei die Nukleinsäure DNA ist.

3. Verfahren nach Anspruch 1 oder 2, wobei die amplifizierte Nukleinsäure aus Schritt (iv) isoliert und/oder analysiert wird.

4. Verfahren nach einem der Ansprüche 1 bis 3, wobei die Nukleinsäureregion, die von Interesse ist, eine Region eines DNA-Moleküls ist, die durch ein Amplifikationsverfahren erzeugt worden ist, wobei das Amplifikationsverfahren bevorzugt PCR ist.

5. Verfahren nach einem der Ansprüche 1 bis 4, wobei die Amplifikationsverfahren der Schritte (ii) und (iv) PCR, NASBA oder Strangverschiebungsamplifikation sind.

6. Verfahren nach einem der Ansprüche 1 bis 5, wobei der Primer 4-60 Nucleotide lang ist, 10-50 Nucleotide lang ist, 15-45 Nucleotide lang ist, 20-40 Nucleotide lang ist oder 25-35 Nucleotide lang ist, wobei bevorzugt der Primer 26, 27, 28, 29, 30, 31, 32, 33 oder 34 Nucleotide lang ist.

7. Verfahren nach einem der Ansprüche 1 bis 6, wobei der Primere durch Folgendes funktionell ineffizient gemacht wird:
(i) Reduzieren der Konzentration von Primer, der verwendet wird;
(ii) Reduzieren der Hybridisierungszeit des Primers;
(iii) Erhöhen der Temperatur, bei der die Hybridisierungsreaktion erfolgen muss;
(iv) Modifizieren der Länge des Primers;
(v) Ändern der Primerschmelztemperatur;
(vi) Einführen des chaotropen Mittels während der Hybridisierungs-/Primerverlängerungsphase;
(vii) Einführen in die Reaktion eines mit dem Primer konkurrierenden Inhibitors, wie beispielsweise seiner komplementären Sequenz;
(viii) Einführen von Nukleotidfehlpaarung in die Primersequenz;
(ix) Verwenden von Primeranalogen, die weniger effizient Wasserstoff im Zusammenhang mit der Hybridisierung binden.

8. Verfahren nach einem der Ansprüche 1 bis 7, wobei die Primergruppe, die ineffizient gemacht worden ist, die Primergruppe ist, die das Potential besitzt, promisk an Nicht-Target-DNA-Regionen zu hybridisieren, wobei bevorzugt der Primer ein degenerierter Primer ist.

9. Verfahren nach einem der Ansprüche 1 bis 8, wobei die Region, die von Interesse ist, eine Region von genomischer DNA, ein Gen, Teil eines Gens, ein DNA-Rekombinationsprodukt oder eine chromosomale Gentranslokationsbruchstelle ist.

10. Verfahren nach Anspruch 9, wobei die chromosomale Gentranlokationsbruchstelle BCR-ABL ist, wobei bevorzugt 1-20 auf BCR gerichtete Vorwärtsprimer und 250-350 auf ABL gerichtete Umkehrprimer verwendet werden.

11. Verfahren nach Anspruch 9, wobei die chromosomale Gentranlokationsbruchstelle PML-RARα ist.

12. Verfahren nach Anspruch 10, wobei 12 auf BCR gerichtete Vorwärtsprimer und 282 auf ABL gerichtete Rückwärtsprimer verwendet werden.

13. Verfahren nach Anspruch 10, wobei 6 auf BCR gerichtete Vorwärtsprimer und 24 auf ABL gerichtete Rückwärtsprimer verwendet werden.

14. Verfahren nach Anspruch 10, wobei 1 auf BCR gerichteter Vorwärtsprimer und 282 auf ABL gerichtete Rückwärtsprimer verwendet werden.

15. Verfahren nach Anspruch 10, wobei 6 auf BCR gerichtete Vorwärtsprimer und 270-310 auf ABL gerichtete Rückwärtsprimer verwendet werden.

## Revendications

1. Procédé d'amplification d'une région d'acide nucléique d'intérêt, ledit procédé comprenant les étapes consistant à :
(i) mettre en contact un échantillon d'acide nucléique avec :
(a) une ou plusieurs amorces directes dirigées sur ladite région d'intérêt ; et
(b) une ou plusieurs amorces inverses dirigées vers ladite région d'intérêt,
dans lequel les amorces de l'un du groupe (a) ou du groupe (b) sont fonctionnellement inefficaces dans la réaction d'amplification de l'étape (ii) et sont liées en service à leur extrémité 5' à une étiquette d'oligonucléotide et dans lequel les amorces de l'autre du groupe (a) ou du groupe (b) sont efficaces dans l'amplification de l'étape (ii) ;
(ii) amplifier l'échantillon d'acide nucléique de l'étape (i) à travers au moins deux cycles d'amplification utilisant des amorces (a) et (b), dans lequel les amorces étiquetées de (a) ou (b) s'hybrident et s'étendent de manière inefficace et les autres amorces de (a) ou (b) s'hybrident et s'étendent de manière efficace ;
(iii) mettre en contact l'acide nucléique amplifié de l'étape (ii) avec une troisième amorce qui est dirigée sur une partie ou la totalité de la séquence qui est complémentaire de celle de l'étiquette d'oligonucléotide de l'étape (i), dans lequel ladite troisième amorce est efficace dans l'amplification de l'étape (iv) ; et
(iv) amplifier l'échantillon d'acide nucléique de l'étape (iii) en utilisant la troisième amorce de l'étape (iii) et les amorces efficaces du groupe (a) ou du groupe (b) de l'étape (i) et de l'étape (ii), dans lequel ladite troisième amorce et les amorces efficaces du groupe (a) ou du groupe (b) s'hybrident et s'étendent de manière efficace.

2. Procédé selon la revendication 1, dans lequel ledit acide nucléique est l'ADN.

3. Procédé selon la revendication 1 ou la revendication 2, dans lequel ledit acide nucléique amplifié de l'étape (iv) est isolé et/ou analysé.

4. Procédé selon l'une quelconque des revendications 1 à 3, dans lequel ladite région d'acide nucléique d'intérêt est une région d'une molécule d'ADN qui a été générée par un procédé d'amplification, de préférence dans lequel ledit procédé d'amplification est la PCR.

5. Procédé selon l'une quelconque des revendications 1 à 4, dans lequel les procédés d'amplification des étapes (ii) et (iv) sont la PCR, la NASBA ou l'amplification par déplacement de brins.

6. Procédé selon l'une quelconque des revendications 1 à 5, dans lequel ladite amorce a une longueur de 4 à 60 nucléotides, une longueur de 10 à 50 nucléotides, une longueur de 15 à 45 nucléotides, une longueur de 20 à 40 nucléotides ou une longueur de 25 à 35 nucléotides, de préférence dans ladite amorce a une longueur de 26, 27, 28, 29, 30, 31, 32, 33 ou 34 nucléotides.

7. Procédé selon l'une quelconque des revendications 1 à 6, dans lequel ladite amorce est rendue fonctionnellement inefficace par :
(i) réduction de la concentration de l'amorce qui est utilisée ;
(ii) réduction du temps d'hybridation de l'amorce ;
(iii) augmentation de la température à laquelle la réaction d'hybridation est censée se produire ;
(iv) modification de la longueur de l'amorce ;
(v) modification de la température de fusion de l'amorce ;
(vi) introduction d'un agent chaotropique au cours de la phase d'hybridation et d'extension de l'amorce ;
(vii) introduction dans la réaction d'un inhibiteur compétitif à l'amorce, notamment sa séquence complémentaire ;
(viii) introduction de discordances nucléotidiques dans la séquence d'amorce ;
(ix) utilisation d'analogues d'amorces qui se lient à l'hydrogène moins efficacement dans le contexte de l'hybridation.

8. Procédé selon l'une quelconque des revendications 1 à 7, dans lequel le groupe d'amorces qui est rendu inefficace est le groupe d'amorces qui a le potentiel de s'hybrider en partenariat multiple avec des régions d'ADN non ciblées, de préférence dans lequel ladite amorce est une amorce dégénérée.

9. Procédé selon l'une quelconque des revendications 1 à 8, dans lequel ladite région d'intérêt est une région d'ADN génomique, un gène, une partie d'un gène, un produit de recombinaison d'ADN ou un point de rupture de translocation de gène chromosomique.

10. Procédé selon la revendication 9, dans lequel ledit point de rupture de translocation de gène chromosomique est le BCR-ABL, de préférence dans lequel on utilise 1 à 20 amorces directes dirigées sur le BCR et 250 à 350 amorces inverses dirigées sur l'ABL.

11. Procédé selon la revendication 9, dans lequel ledit point de rupture de translocation de gène chromosomique est le PML-RARα.

12. Procédé selon la revendication 10, dans lequel on utilise 12 amorces directes dirigées sur le BCR et 282 amorces inverses dirigées sur l'ABL.

13. Procédé selon la revendication 10, dans lequel on utilise 6 amorces directes dirigées sur le BCR et 24 amorces inverses dirigées sur l'ABL.

14. Procédé selon la revendication 10, dans lequel on utilise 1 amorce directe dirigée sur le BCR et 282 amorces inverses dirigées sur l'ABL.

15. Procédé selon la revendication 10, dans lequel on utilise 6 amorces directes dirigées sur le BCR et 270 à 310 amorces inverses dirigées sur l'ABL.
